(19) Europäisches Patentamt / European Patent Office / Office européen des brevets

(11) **EP 3 993 858 B1**

(12) **EUROPEAN PATENT SPECIFICATION**

(45) Date of publication and mention
of the grant of the patent:
**21.08.2024 Bulletin 2024/34**

(21) Application number: **20754180.6**

(22) Date of filing: **28.07.2020**

(51) International Patent Classification (IPC):
*A61M 15/00* *(2006.01)*  *A61M 16/00* *(2006.01)*
*A61B 5/00* *(2006.01)*  *G01F 1/00* *(2022.01)*
*A61B 5/087* *(2006.01)*  *G01F 1/34* *(2006.01)*
*G01F 1/74* *(2006.01)*  *G01F 15/075* *(2006.01)*

(52) Cooperative Patent Classification (CPC):
**A61M 15/0021; A61B 5/0022; A61B 5/087;
A61B 5/4839; A61B 5/7455; A61M 15/008;
A61M 15/0086; G01F 1/34; G01F 1/74;
G01F 15/075;** A61B 2562/0247; A61M 15/003;
A61M 15/009; A61M 2016/0021; A61M 2016/0027;

(Cont.)

(86) International application number:
**PCT/EP2020/071222**

(87) International publication number:
**WO 2021/037462 (04.03.2021 Gazette 2021/09)**

(54) **DOSE MEASURING DEVICE AND METHOD IN INHALERS**

VORRICHTUNG UND VERFAHREN ZUR DOSISMESSUNG BEI INHALATOREN

DISPOSITIF ET PROCÉDÉ DE MESURE DE DOSE DANS DES INHALATEURS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.08.2019 ES 201930752**

(43) Date of publication of application:
**11.05.2022 Bulletin 2022/19**

(73) Proprietor: **IGNCYERTO S.L.**
**28020 Madrid (ES)**

(72) Inventors:
• **CALVO FERNÁNDEZ, Alberto**
**28020 Madrid (ES)**
• **ESTEBAN GORGOJO, Ignacio**
**28020 Madrid (ES)**
• **LAGUNA GALARZA, Eduardo**
**28020 Madrid (ES)**

(74) Representative: **Clarke, Modet y Cía., S.L.**
**C/ Suero de Quiñones 34-36
28002 Madrid (ES)**

(56) References cited:
**WO-A1-2011/157561    WO-A1-2016/030521
WO-A1-2016/033421    WO-A1-2018/160073
WO-A1-2018/167278    WO-A1-2018/175579
GB-A- 2 294 402    US-A1- 2009 314 292
US-A1- 2018 161 531**

(52) Cooperative Patent Classification (CPC): (Cont.)
A61M 2016/0039; A61M 2205/13; A61M 2205/3313;
A61M 2205/3317; A61M 2205/332;
A61M 2205/3584; A61M 2205/505; A61M 2205/52;
A61M 2205/8212

## Description

### Field of the invention

The invention is comprised in the field of biotechnology and smart devices. In this case, the device and method designed complement the use of dry powder inhalers and pressurized inhalers using aerosolized medication.

### Background of the invention

A dry powder inhaler is a medicinal product delivering a medicinal product to the patient in the form of powder particles through the lungs, by means of inhalation. A pressurized inhaler or MDI (metered-dose inhaler) is a medicinal product delivering a medicinal product to the patient by means of spraying said product in an aerosol, releasing a specific amount of medication that is always constant in each inhalation. The medication is thereby suspended in the inhaled air and reaches the lungs. These inhalers are primarily used for the treatment of asthma and chronic obstructive pulmonary disease (COPD), but their use today is spreading to more pathologies.

At present, fewer than 30 % of patients with asthma or COPD properly adhere to treatment according to the scientific literature. In addition to the lack of adherence, these devices are known to be hard to use, so there are errors in the inhaling technique, estimating that such technique is correct in 10% of the patients who use them. There is currently no validated tool for assessing same. When analyzing these two parts of data and relating them to one another, it can be estimated that fewer than 3 % of patients who use these devices are receiving the amount of medication prescribed by their doctor in the target organ, the lung.

This situation converts the lack of adherence and inhaling technique errors into the highest causes of poorly controlling pulmonary diseases. For doctors to correctly follow up on treatment, it has become necessary to detect the intentional group, where patients deliberately do not take medicinal products. However, it is also important to detect the occurrence of the two non-intentional scenarios, which are involuntary oversight and the aforementioned errors in the technique. Once the three causes of non-compliance have been taken into account and measured, it is then possible to begin obtaining real information about managing the disease and its true repercussion.

It is also important to point out that the pressurized MDI devices are the most inexpensive inhalers existing on the market and the most widely distributed worldwide, unlike dry powder inhalers which, due to their high price, are more restricted in use to more developed countries, where the total invoicing for both is similar on a global level. MDI devices are the most widely used for the administration of salvage medication, which is used when the patient perceives symptoms or a flare-up, whereas dry powder inhalers are the most widely used for maintenance treatments. Therefore, being able to develop a technology that can help improve adherence and the technique of using these inhalers, both dry powder inhalers and MDIs, has a worldwide impact and affects the evaluation of both maintenance treatment and salvage therapy.

The present invention solves this problem and allows preventing the involuntary lack of adherence to inhalation treatments (involuntary oversight and poor technique), both with dry powder inhalers and with the pressurized inhalers, and bringing the intentional lack of adherence to the forefront. Optimizing adherence to inhaled medication leads to better control of pulmonary diseases, with patients having better control of their disease and with an objective measuring of salvage medication. This should reduce visits to emergency rooms and hospital admissions, in addition to the fact that it should eliminate or mitigate the limitations to patients' daily activities.

Correct monitoring of inhalation treatments will allow the best inhalation device to be prescribed to each patient, thereby optimizing the prescribed treatment. This correct choice of the device that is suitable for each patient potentiates customized medicine and allows overdose and overprescribing medication that is not reaching the target organ to be avoided, and, therefore adverse effects can increase without increasing the therapeutic benefit.

Finally, this technology is a cornerstone for controlling and reducing spending on respiratory diseases. Between 70 % and 80 % of spending for asthma and COPD is derived from performing tests, follow-up in consultations, prescription of medication and visits to emergency rooms and hospital admissions. This technology may reduce all these costs simply with the improvement and the demonstration that the prescribed medication is taken. Spending for these diseases can thereby be optimized, drawing it away from avoidable factors towards necessary and unavoidable factors.

Document WO2018175579-A1 discloses a dry powder inhaler that estimates the mass of powder medication delivered to a patient during an inhalation using an optical sensor and a formula stored in memory obtained from a complex calibration.

### Description of the invention

The invention relates to a device and a method for dose measurement in inhalers, which is capable of calculating the dose of solid particles dispensed by the inhaler in each inhalation (the dose that would reach the mouth of the user). The invention allows measuring adherence to an inhalation treatment and estimating the amount of inhaled medication which is really distributed in the lungs, and thereby allowing the inhalation technique and the use of the inhaler by the patient to be evaluated.

The device is coupled to a dry powder inhaler or to a pressurized inhaler, being adapted to the structure and shape of said inhaler. Once it has been coupled, the device allows counting the number of real inhaled doses, which may differ from the doses indicated in the dose indicator of the inhaler itself. Furthermore, the device quantifies the dose dispensed by the inhaler and, after analyzing the quality of the inhalation technique, estimating how much medication is distributed in the lung, the only organ in which it would produce the desired effect.

The dose measuring device for an inhaler comprises:

- An inhalation mouthpiece, comprising an outlet conduit for the passage of inhaled air.
- A powder sensor configured for measuring the density of solid particles suspended in air inside the outlet conduit.
- A barometric pressure sensor for measuring the pressure of the air inside the outlet conduit.
- A control unit configured for detecting an inhalation produced in the inhalation mouthpiece by means of analyzing the pressure of the air inside the outlet conduit; and calculating, using the combined information of the powder sensor and the pressure signal of the barometric pressure sensor, a dose of solid particles suspended in the inhaled air going through the outlet conduit in the detected inhalation, wherein for calculating the dose of solid particles going through the outlet conduit in a period of time, the control unit is configured for:
- determining, by means of the reading of the powder sensor, the density of solid particles inside the outlet conduit in a plurality of sampling instants during the period of time;
- calculating the airflow going through the outlet conduit in said plurality of sampling instants using the pressure signal of the barometric pressure sensor;
- determining the mass flow rate of solid particles going through the outlet conduit in said plurality of sampling instants; and
- integrating the mass flow rate in said period of time.

The dose measuring method in inhalers comprises the following steps:

- Measuring, by means of a barometric pressure sensor, the pressure of the air inside an outlet conduit of an inhalation mouthpiece.
- Detecting an inhalation by means of analyzing the pressure of the air inside the outlet conduit.
- Measuring, by means of a powder sensor, the density of solid particles suspended in air inside the outlet conduit.
- Calculating, using the combined information of the powder sensor and of the barometric pressure sensor, a dose of solid particles suspended in the inhaled air going through the outlet conduit in the detected inhalation, wherein for calculating the dose of solid particles going through the outlet conduit in a period of time, the control unit is configured for:

- determining, by means of the reading of the powder sensor, the density of solid particles inside the outlet conduit in a plurality of sampling instants during the period of time;
- calculating the airflow going through the outlet conduit in said plurality of sampling instants using the pressure signal of the barometric pressure sensor;
- determining the mass flow rate of solid particles going through the outlet conduit in said plurality of sampling instants; and
- integrating the mass flow rate in said period of time.

One of the functions of the device is to ensure that the patient performs inhalation, with sensors to demonstrate that the patient has the inhaler or spacing chamber in the mouth, and furthermore analyzing the inhalation technique to enable predicting the real distribution of medication in the lung, and therefore to enable determining the real and active dose of the drug the patient has received in each inhalation.

The device may comprise the following elements:

- Plastic casing adaptable to each device housing electronic elements and/or modules and adapted to each inhaler or spacing chamber.
- Inhalation mouthpiece which is coupled to the free end of the mouthpiece of an inhaler or of a spacing chamber.
- Pressure, light or impedance detection sensors coupled to the outer face of the mouthpiece.
- DC/DC converter: Voltage booster and/or reducing device to provide the operating voltage needed for the remaining electronic elements.
- Bluetooth micromodule for wireless communication with external devices (smart phones, computers, etc.).

For measuring inhalation, the device consists of an optical powder sensor assembled in an inhalation mouthpiece which continues to the original outlet of the inhaler. This mouthpiece, with a nozzle geometer, assembles an infrared LED and a serial receiver with a signal amplifier which generates a pulse pattern based on periodically checking the output voltage, for example every 10 ms. This output pattern is interpreted by an algorithm programmed in the motherboard of the device. The inhalation reading is activated when a pre-established variation of pressures in the mouthpiece is detected, using the signal of the digital barometer. The powder sensor measures the passage of medicinal product through the inhalation mouthpiece and allows the control unit to estimate the amount of medication released, the time it is released and the duration of the release of this medication.

The barometric pressure sensor is used, among other functions, to enable analyzing the inhalation technique. The technique for correctly performing inhalation depends on the intensity of the inhalation airflow and the temporary relationship between the intensity of the inhalation flow and the release of the medication. Furthermore, this detected pressure signal is used as an activation signal of other electronic components (e.g., for activating the powder sensor and the control unit), with the subsequent energy savings. The digital barometer allows discriminating whether the user is correctly performing inhalation and predicting how much medication is distributed in the lung.

The device can be connected by means of a wireless communication module, preferably by Bluetooth or IoT signaling, with external apparatuses, such as mobile devices (tablets, smartphones, etc.) and computers. Through a software application installed in said apparatus, the history of inhalations performed and the history of inhaled doses, in addition to other additional data such as location, climate, etc., can be recorded.

The device of the present invention can detect when inhalation has occurred, and if the measured inhaled amount is insufficient, alerting the patient (for example, through the app of his or her smartphone connected to the device) of a deficient quality of the inhalation technique. This alert can be generated not only with a specific inhalation, but rather by taking into account the inhaled dose accumulated over the last few days of treatment, thereby assessing not only an isolated technique, but also the treatment as a global and dynamic entity. The app can thereby recommend that the patient visit his or her doctor, nurse or trainer to improve the inhalation technique. The app can also remind the patient that a dose has been forgotten, reinforcing patient adherence to the inhalation treatment established by the doctor. The monitoring history of inhalations performed and of inhaled doses of medicinal product allows the doctor to determine during periodic checkups whether the treatment is followed correctly and even to discover the voluntary lacks of adherence by the patient (for example, in the event that the detected inhalations do not coincide by a wide margin with the dose indicator of the inhaler). Furthermore, by using the information captured by the device doctors can demonstrate the adherence, the technique and the distribution of drugs in the lungs, to enable differentiating if poor control of asthma is due to poor adherence or an insufficient dose, thereby improving the treatment to be followed.

Another aspect provided by this technology is that through presence sensors (pressure, impedance or light sensors placed in the outer portion of the mouthpiece), it can be certified that the patient has his or her mouth correctly positioned on the mouthpiece when inhalation is performed. This is important because inhalations in which the patient is not receiving medication will therefore not be counted. The possibility of pretending that inhalations are being performed when they actually are not is thereby eliminated, because some patients pretend to have taken the medication, but have not really taken it, and by knowing that the doses are monitored, they may discharge into the air those devices that are not operated with inhalation. Inhalation errors related to an incorrect placement of the device in the mouth or poor coupling with a spacing chamber can also be detected. This poor coupling with the mouth or the spacing chamber would significantly alter the pulmonary distribution of the drug, and it is therefore essential to be able to detect such distribution.

Furthermore, since the device allows monitoring that treatment is being followed, patients who intentionally avoid taking their medicinal products are encouraged to submit to the treatment in order to avoid the shame of acknowledging, during the periodic checkup with their doctor, that they have not following the medical advice. In the event that the patient maintains his or her intentional lack of adherence to treatment, the device detects this situation and allows doctors to prevent an overdose or expose patients to unnecessary treatments, such as biological products.

Another aspect of the present invention also relates to an inhaler or a spacing chamber for inhalers comprising the dose measuring device, where the inhalation mouthpiece is an integral part (e.g. integrated during the manufacturing process) of the inhaler or of the spacing chamber for inhalers.

The present invention thereby allows improving adherence to inhalation treatments in diseases such as the asthma and chronic obstructive pulmonary disease.

**Brief description of the drawings**

What follows is a very brief description of a series of drawings that aid in better understanding the invention, and which are expressly related to one embodiment of said invention which is presented by way of non-limiting examples of the same.

Figure 1 shows a generic inhaler from the state of the art to which the device of the present invention can be coupled.
Figures 2A-2E depict several views of a dose measuring device for an inhaler according to an embodiment of the present invention.
Figure 3 illustrates a dose measuring device according to the present invention coupled to an inhaler.
Figures 4A and 4B show several views of the front portion of the inhalation mouthpiece.
Figure 5 illustrates a perspective view of the dose measuring device where the components of the powder sensor installed inside the inhalation mouthpiece can be seen.
Figure 6 shows a diagram of the electronic components of the dose measuring device and their wireless connection with an external mobile device.

Figures 7A, 7B and 7C illustrate, respectively, a side view (Figure 7A), a front view (Figure 7B) and a perspective view (Figure 7B) of an inhaler which incorporates during manufacture the powder sensor and the barometric sensor in the mouthpiece of the inhaler.

Figure 8 shows a diagram of the electronic components of the inhaler of Figure 7 and their wireless connection with an external mobile device.

Figure 9 illustrates a flowchart of a dose measuring method for measuring a dose released by an inhaler according to the present invention.

Figure 10 includes a flowchart which details how the calculation of the dose of solid particles released by the inhaler is performed according to a possible embodiment.

Figures 11A and 11B depict, respectively, a graph of the inspiration produced during inhalation in a dry powder inhaler and a graph used for estimating the distribution of medication in the lung depending on the inspired airflow rate.

Figure 12A shows a graph of the inspiration produced during an inhalation in a pressurized inhaler. Figures 12B and 12C illustrate graphs used for estimating the distribution of medication in the lung depending on the inspired airflow rate after the release of medicinal product (Figure 12B) and before the release of medicinal product (Figure 12C).

Figures 13A and 13B illustrate, respectively, examples of pressurized inhalers with more than one release of medicinal product or with a prolonged release of medicinal product.

Figure 14 depicts a spacing chamber incorporating the dose measuring device installed in the mouthpiece of the spacing chamber.

**Detailed description of the invention**

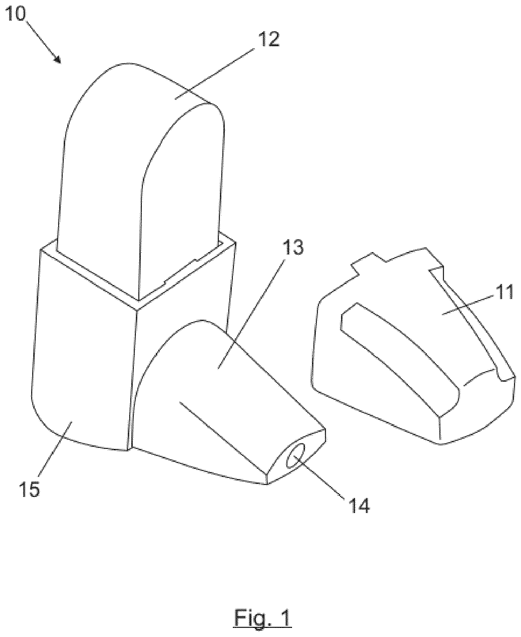

The present invention relates to a dose measuring device for an inhaler, whether a dry powder inhaler, a pressurized inhaler or a spacing chamber. **Figure 1** depicts, by way of example, an inhaler 10 of the state of the art to which the device of the present invention is directed. The inhaler 10 depicted in Figure 1 may correspond to a dry powder inhaler or to a pressurized inhaler.

The usual operation of a dry powder inhaler by a user includes the following steps:

- Removing the cover 11 from the inhaler.
- Loading the dose. In some devices, the dose can be loaded, for example, by shaking the inhaler and pressing the button 12 located in the upper portion of the inhaler when it is in the vertical position. In other devices, it is loaded in a different manner, such as for example by turning a wheel or puncturing a capsule. Other devices are automatically loaded upon removing the cover 11.
- Placing the mouthpiece of the inhaler 13 between the lips and teeth and inspiring vigorously and deeply until the inhaled air circulates through the orifice 14 of the mouthpiece and fills the lungs. In dry powder inhalers, it is the inspiration by the patient that generates the stream for transporting the medicinal product.

The usual operation of a pressurized inhaler by a user includes the following steps:

- Removing the cover from the inhaler 11.
- Shaking the inhaler.
- Placing the mouthpiece of the inhaler 13 between the lips and teeth and inspiring continuously starting a second before pressing the button 12 located in the upper portion of the inhaler when it is in the vertical position, until the inhaled air circulates through the orifice 14 of the mouthpiece and fills the lungs.

The usual operation of a spacing chamber by a user includes the following steps:

- Coupling the pressurized inhaler to an end of the spacing chamber adaptable for inhalers.
- Coupling the lips to the opposite end of the chamber. Placing the mouthpiece of the spacing chamber between the lips and teeth.
- Releasing the medication from the inhaler and performing 10 normal, unforced inspiration cycles. Sometimes single forced inhalation until the lungs are filled may also be performed.

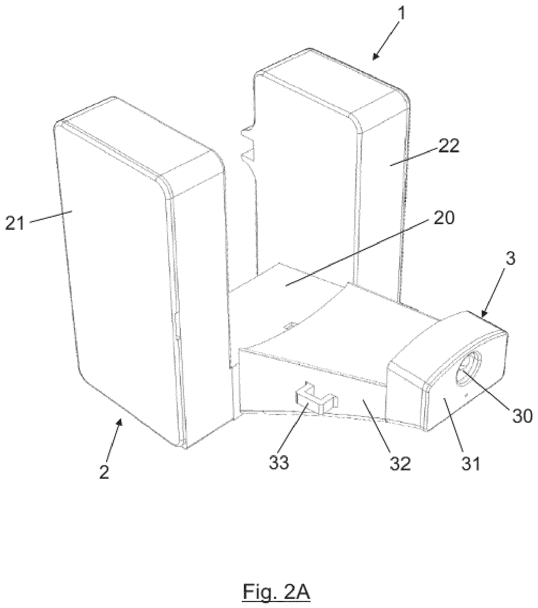

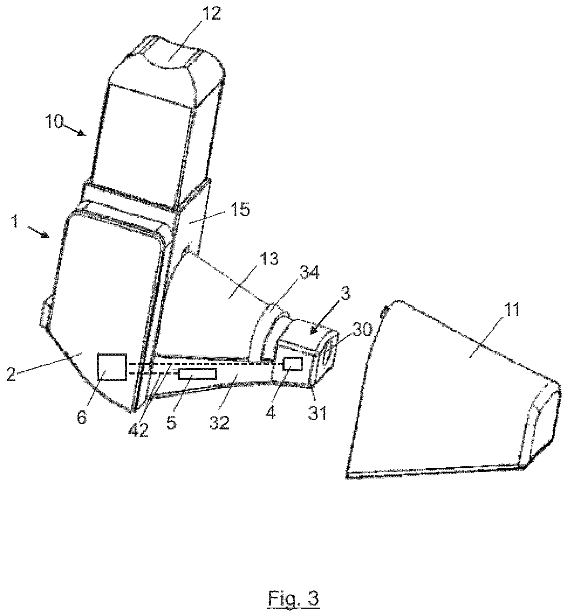

**Figures 2A-2E** respectively show a perspective view, a front view, a profile view, a top view and a rear view of a dose measuring device 1 for a (dry powder or pressurized) inhaler according to a possible embodiment of the present invention. The dose measuring device 1 is prepared for being coupled to a generic dry powder and pressurized inhaler 10, such as the one depicted in Figure 1 for example. **Figure 3** depicts a dose measuring device 1 (in this case corresponding to another possible embodiment with slight changes in the shape of the casing 2 and in the securing means) already coupled to an inhaler 10.

The dose measuring device 1 for an inhaler comprises a comprises an inhalation mouthpiece 3 which is coupled, for example by means of pressure or using securing means, to the free end of the mouthpiece 13 of an inhaler, as shown in the example of Figure 3. This inhalation mouthpiece 3 will be what contacts, at least at one of its ends, the mouth of the user at the time of performing the inhalation. The inhaled air passes to the mouth of the user through a first air passage orifice 30 made in the front portion of the inhalation mouthpiece 3. Said first inhaled air passage orifice 30 preferably has a size and position such that, once it is coupled to the mouthpiece of the inhaler 13, it coincides at least partially (in a front view) with the orifice 14 of the mouthpiece of the inhaler 13 to facilitate circulation of the inspired air.

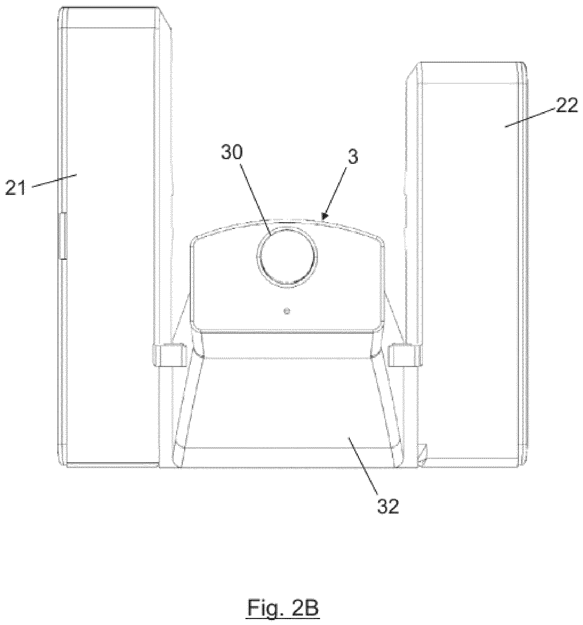

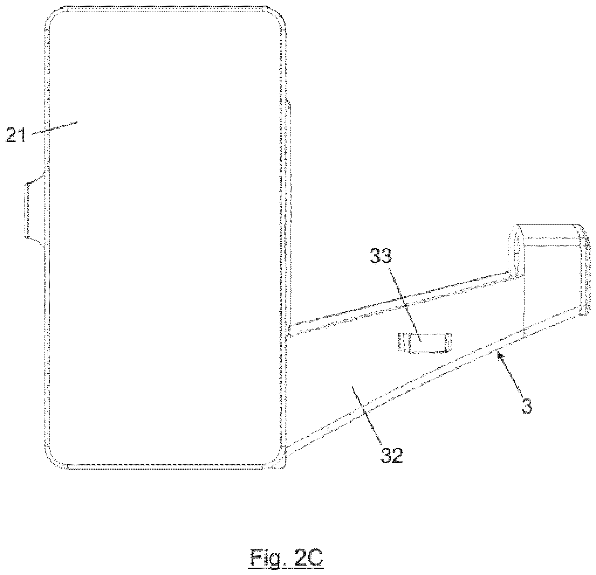

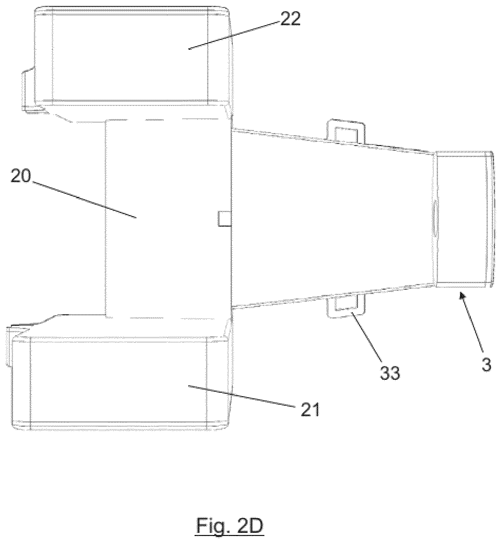

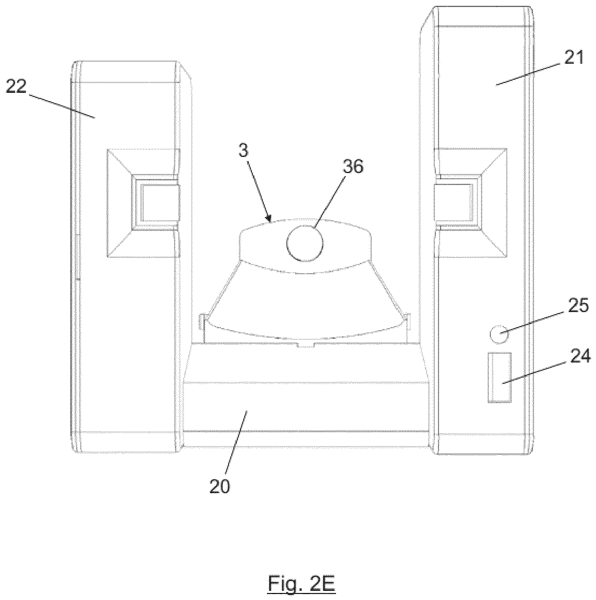

Although the inhalation mouthpiece 3 is coupleable to the free end of the mouthpiece 13 of an inhaler 10 in the example depicted in Figures 2 and 3, in another possible embodiment the inhalation mouthpiece 3 is coupleable to the free end of the mouthpiece of a spacing chamber for inhalers.

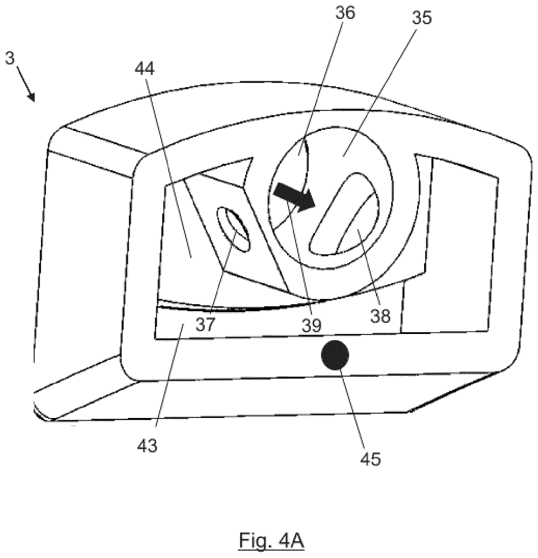

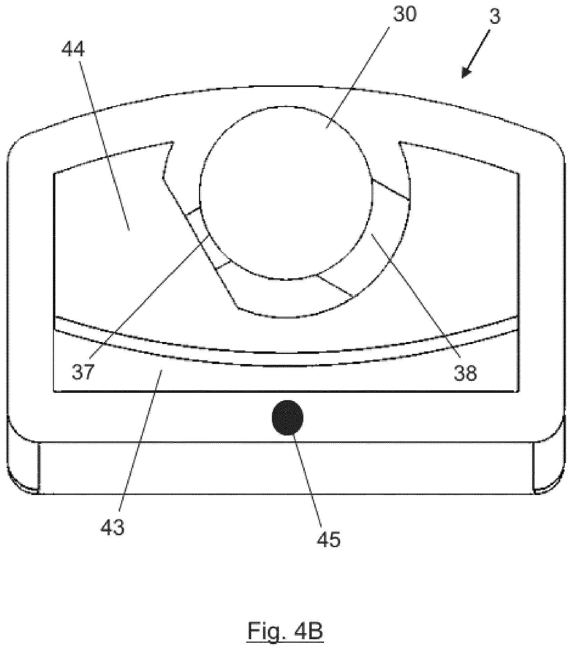

**Figures 4A** and **4B** respectively illustrate a perspective view and a front view of the front end of the inhalation mouthpiece 3 in which a front cover 31 has been removed in order to see in detail the inside of the mouthpiece. The air inhaled 39 by the user, in which a dose of medicinal product coming from the inhaler 10 flows, exits through the orifice 14 of the mouthpiece of the inhaler 13, enters the inside of the inhalation mouthpiece 3 through a second orifice 36 made in its rear portion (which coincides at least partially with the orifice 14 of the mouthpiece of the inhaler 13), circulates through a conduit 35 inside the inhalation mouthpiece 3 and exits through the first inhaled air passage orifice 30 made in the front portion of the inhalation mouthpiece 3. To guarantee that all the inhaled air 39 circulating through the conduit 35 passes through the orifice 30, the conduit 35 is preferably in contact with the first orifice 30, such that the first orifice 30 may be regarded as the front end of the conduit 35 or as a continuation of the conduit 35 with an identical or different area or section. In a possible embodiment, the conduit 35 may have a very small thickness, coinciding for example with the thickness of the front cover 31 and with the first orifice 30. The first orifice 30 and second orifice 36 of the inhalation mouthpiece 3 are preferably circular-shaped, although they may adopt other shapes. Similarly, the conduit 35 of the inhalation mouthpiece 3 preferably has a circular section, although it may adopt sections of another type (e.g., square).

The dose measuring device 1 comprises a powder sensor 4 integrated inside the inhalation mouthpiece 3. The powder sensor 4 is configured for detecting the presence of solid particles suspended in air inside the conduit 35 of the inhalation mouthpiece 3. Preferably, the powder sensor 4 used is an optical powder sensor. The optical powder sensors comprise an optical emitter and an optical receiver. The optical emitter is preferably a light emitting diode in the infrared spectrum (infrared LED or IRED). The optical receiver can be a photodiode or a phototransistor, which detects the infrared light reflected by the powder or solid particles suspended in air. By means of the reading of the powder sensor 4, the powder density in the inspired airflow can be calculated.

The dose measuring device 1 may also comprise one or several presence sensors 45 installed on the outer face of the inhalation mouthpiece 3, through which the control unit 6 detects contact of the inhalation mouthpiece with the mouth of a user. The control unit 6 records the detected inhalation only when all the presence sensors are activated and detect contact of the mouth of a user during the inhalation.

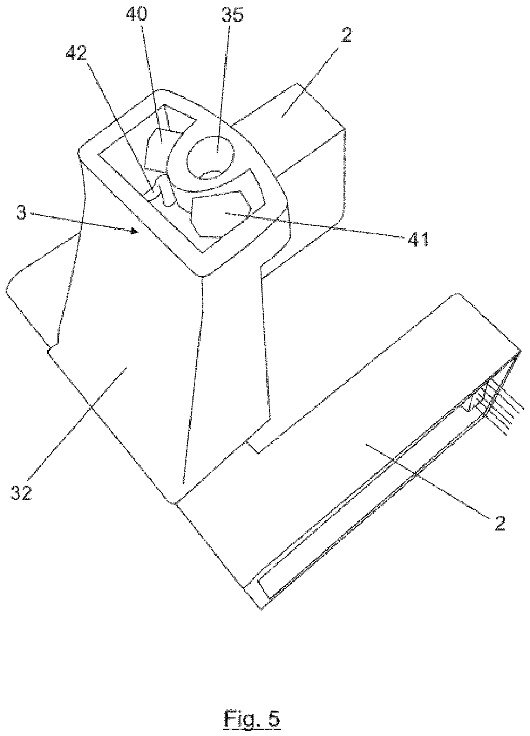

According to the embodiment shown in **Figures 4** and **5,** the optical emitter 40 of the powder sensor 4 is located outside the conduit 35, next to a first opening 37 made therein, which allows the passage of the beam of preferably infrared light coming from the optical emitter 40. Similarly, the optical receiver 41 of the powder sensor 4 is arranged adjacent to a second opening 38 made in the conduit 35, which allows receiving the beam of infrared light reflected by the powder or solid particles (i.e. the medicine coming from the inhaler 10) suspended in the inhaled air 39 circulating through the conduit 35. The openings (37, 38) are located in the conduit 35 preferably facing one another (in the example of the figure the vectors perpendicular to the openings at their midpoints are focused on one and the same point of the longitudinal axis of the conduit 35, to obtain a better reading).

The dose measuring device 1 also comprises a barometric pressure sensor 5 integrated in the inhalation mouthpiece 3 and configured for measuring the pressure of the air inside the outlet conduit 35 of the inhalation mouthpiece 3.

In one embodiment, the inhalation mouthpiece 3 comprises only the front end part depicted in Figure 4 which, in addition to housing the powder sensor 4, would also include the barometric pressure sensor 5. In the embodiment shown in Figures 2 and 3, the inhalation mouthpiece 3 comprises, in addition to the front part depicted in Figure 4, a hollow lower part 32 which is coupled to said front part. Alternatively, the front part and the lower part are formed in a single part.

According to one embodiment, the barometric pressure sensor 5 is housed in the lower portion of the inhalation mouthpiece 3 (housed for example in the lower part 32, as shown with a discontinuous line in Figure 3), which is hollow and allows, in addition to housing said sensor, the passage of wiring 42 for connecting the powder sensor 4 with a control unit 6 housed in a casing 2 of the dose measuring device 1 (in this case, the wiring 42 of the powder sensor 4 would pass through an opening 43 below a rear plate 44 containing the second orifice 36 of the front part of the inhalation mouthpiece 3).

The barometric pressure sensor 5 is housed inside the inhalation mouthpiece 3 in contact with the inhaled air 39 circulating through the conduit 35. For example, it could be housed in an opening made in the actual conduit 35, similarly to how the optical emitter 40 and optical receiver 41 of the powder sensor 4 are housed (Figure 5). In the example of Figure 3, in which the barometric pressure sensor 5 is housed in the lower part 32 of the inhalation mouthpiece 3, for measuring the pressure of the air circulating through the inner of the conduit 35, the conduit 35 must not be isolated from the lower part 32; that is, the conduit 35 and the lower part 32 where the barometric pressure sensor 5 is housed must be in aerial contact, at the same barometric pressure. To that end, one or both ends of the conduit 35 can be free, or the conduit 35 may have an orifice which allows aerial contact with the hollow lower part 32. In the example of Figure 4, the back end of the conduit 35 does not contact the rear plate 44 of the inhalation mouthpiece 3.

The dose measuring device 1 comprises a control unit 6 (a circuit or hardware element with electronic control functions, implemented for example by means of a microcontroller), configured for detecting an inhalation produced in the inhaler 10 by means of analyzing the pressure of the air inside the inhalation mouthpiece 3, using the reading of the barometric pressure sensor 5. Once the inhalation has been detected, the control unit 6 is in charge of estimating the dose of solid particles suspended in air going through the inhalation mouthpiece 3 in said inhalation, using the reading of the powder sensor 4.

The control unit 6 can be housed in the actual inhalation mouthpiece 3, for example in the lower part 32. Alternatively, the control unit 6 can be housed in a casing 2 attached to the inhalation mouthpiece 3, as shown in Figures 2 and 3. In this case, the control unit 6 can be connected to the optical sensor 40 and to the barometric pressure sensor 5 by means of wiring 42 running inside the inhalation mouthpiece 3 up to the inside of the casing 2.

The casing 2 shown in Figures 2 and 3 comprises a base 20 and two side parts (21, 22) which are prepared for receiving and/or housing the body 15 of the inhaler 10 (the body 15 is the portion of the inhaler that does not include the mouthpiece 13 or the button 12). The lower part 32 of the inhalation mouthpiece 3 is connected to the base 20 of the casing 2 and has a shape which allows at least partially housing or supporting the mouthpiece of the inhaler 13. The inhaler 10 can thereby be readily fitted in the dose measuring device 1 by means of a vertical insertion movement. To facilitate the fixing of the inhaler 10, the dose measuring device 1 may comprise securing means arranged in the casing 2 and/or in the inhalation mouthpiece 3, such as side rings 33 located in the inhalation mouthpiece 3 for introducing a tightening band or rubber, or a securing strip 34 integral with the actual inhalation mouthpiece 3.

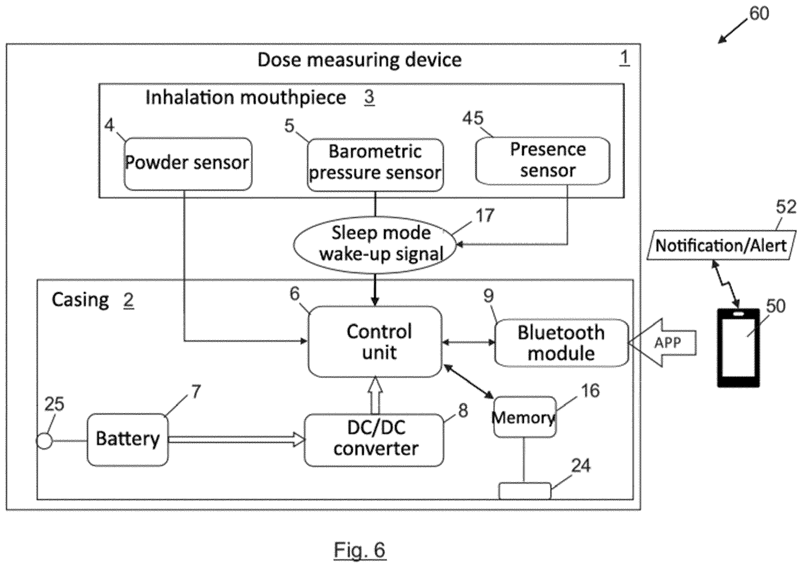

The casing 2 can be manufactured in a single part or may be made up of several individual parts attached to one another. The inside of the casing 2 can house different electronic components, the control unit 6 being among such components. **Figure 6** depicts a block diagram with the electronic components of the dose measuring device 1 according to a possible embodiment. A battery 7 housed in a casing supplies energy to the control unit 6 through a DC/DC converter 8 (for example, a 5V boost converter) to ensure a voltage level that is constant and determined at the control unit 6. The control unit 6 (or alternatively the actual battery 7) supplies power to the powder sensor 4 and the barometric pressure sensor 5. The battery 7 is preferably as rechargeable battery, and it can be charged through a charging connector 25 located in the rear portion of the casing (Figure 2E). The device may include a battery charging circuit.

A wireless communication module 9 (implemented for example by means of a Bluetooth module or a Wi-Fi module) allows the remote connection to a control station (e.g., a computer) or a mobile device 50 (e.g., a smart phone) for transmitting information calculated and/or compiled by the control unit 6. For example, the control unit 6 can store in an internal memory a history of inhalations performed and measurements of inhaled doses, which are transmitted through the Bluetooth module to an application of a mobile device 50 connected by means of Bluetooth.

The dose measuring device 1 and mobile device 50 assembly can form a dose measuring system 60, wherein the dose measuring device 1 performs the measurement of the inhalations performed and the doses inhaled, and the mobile device 50 monitors said data and can send notifications and/or alerts 52 informing about the compliance or non-compliance with the established inhalation treatment. The notifications and/or alerts 52 can be communicated locally to the patient or user of the mobile device 50, for example through information or messages shown on the display of the mobile device 50, sound messages, acoustic or vibrational alarms, etc. The information relative to compliance or non-compliance with the inhalation treatment can also be communicated to a remote device, for example to another mobile device or a computer through an Internet connection, whereby other people (relatives of the patient, doctor, nurse, etc.) may have access to the data captured by the dose measuring device 1.

Alternatively or additionally, the device may include a memory 16 (such as an EEPROM memory, a flash memory, a micro SD card, etc.), which can be incorporated in the actual control unit 6. The information stored in the memory 16 can be accessed through a communication port 24 (for example, a USB, mini USB or micro USB port) located on the outside of the casing 2. In the event that a memory card (e.g., a micro SD card) is used for storing the information, the device allows directly retrieving the memory card, for example by means of activating a button.

For energy savings of the battery 7, the control unit 6 (for example a microcontroller) can operate in energy saving mode or sleep mode most of the time and use the pressure signal coming from the barometric pressure sensor 5 to wake up the microcontroller from the sleep mode, similarly to an interruption. For example, when the pressure signal exceeds a given threshold, the control unit 6 wakes up from the sleep mode (signal 17 in Figure 6). In the event that the inhalation mouthpiece 3 incorporates a presence sensor 45, the sleep mode wake-up signal 17 can be generated additionally when the presence sensor 45 is activated.

When the user or patient performs an inhalation, the barometric pressure sensor 5 is activated and measures the differential pressure between the aspiration pressure and atmospheric pressure. When the medication passes through the inhalation mouthpiece 3 of the device, the powder sensor 4 detects the passage of particles and provides a reading of the powder density in the aspired flow. The level of the output voltage of the powder sensor 4 determines the powder density, according to a graph or function which depends on the particular powder sensor 4 used. For example, a 3V output of a commercial powder sensor 4 may signify a powder density of 0.4 mg/m³.

The inhaled air flow rate $Q$ circulating through the conduit 35 of the inhalation mouthpiece 3 is determined on the basis of the rate of passage $V_d$ of the fluid through the section of the conduit $S_d$.

$$Q = V_d S_d = S_d \sqrt{\frac{2\left(p_a^+ - p_d^+\right)}{(1-\beta^4)}}$$

The primary equation determining the rate of passage $V_d$ of the aspiration is as follows:

$$V_d = CY \sqrt{\frac{2\rho_a\left(p_a^+ - p_d^+\right)}{\rho_d^2(1-\beta^4)}}$$

Where C is the discharge coefficient, the material balance constant $\beta$ is obtained experimentally (the value is comprised between 0.2 and 0.75, being an adimensional variable which can initially be set at 0.4), and the expansion factor Y is calculated according to the following expression:

$$Y = 1 - \frac{p_a - p_d}{p_a \gamma}(0.41 + 0.35\beta^4)$$

It can be assumed that the adiabatic coefficient $\gamma$ has a value of 1.4 on the basis of the following variability range:

$$1.1 < \gamma < 1.5$$

In the preceding equations, it can be assumed that $P_a$ is the atmospheric pressure and $P_d$ is the pressure read by the barometric pressure sensor 5. The rate of passage $V_d$ of the airflow, and with it the flow rate of the fluid $Q$, is thereby characterized.

With the combined information of the aspiration flow rate (in m³/s, obtained continuously during the inspiration through the pressure signal of the barometric pressure sensor 5) and the powder density (in mg/m³, obtained in each instant through the output signal of the powder sensor 4), the mass flow rate of solid particles (in mg/s) circulating through the conduit 35 in each instant is determined. By integrating said mass flow rate during the time the inhalation lasts, the mass of solid particles (in mg) inhaled is obtained and can be converted directly into the medicinal product active ingredient dose that the patient has inhaled, depending on the ratio between the active ingredient and excipients of each particular medicinal product used.

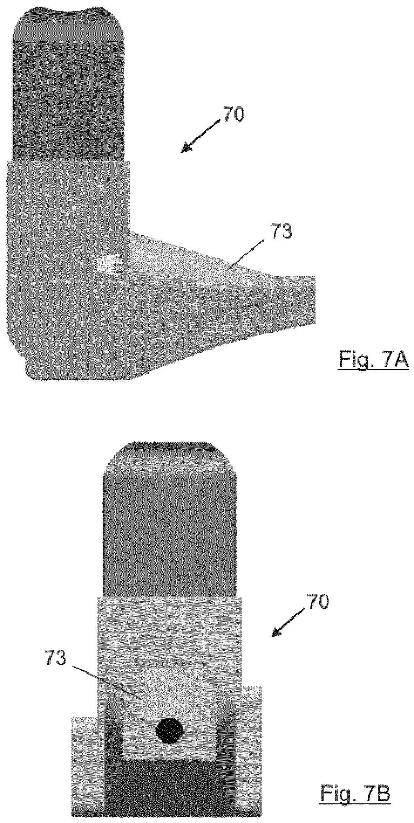

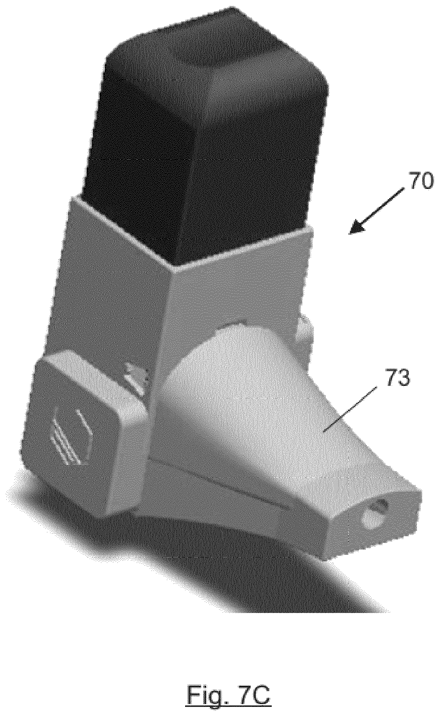

**Figures 7A, 7B** and **7C** depict different views of a (dry powder or pressurized) inhaler 70 which incorporates during manufacture the powder sensor 4 and the barometric pressure sensor 5 already installed in the mouthpiece 73 of the inhaler. In this case, the inhalation mouthpiece 73 is therefore an integral part of an inhaler 70.

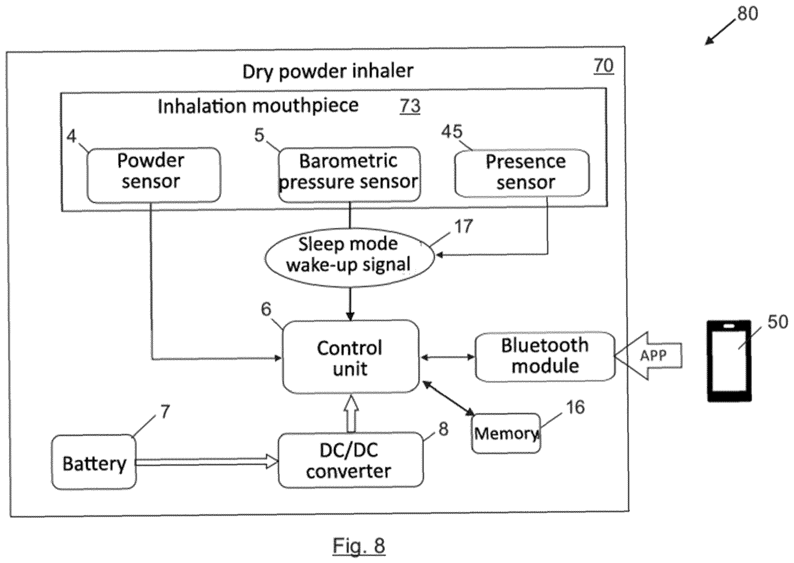

**Figure 8** depicts a figure equivalent to Figure 6, in which the dose measuring device 1 has basically been replaced with the inhaler 70 of Figure 7. The mobile device 50 and inhaler 70 assembly may also form a dose measuring system 80, as explained in Figure 6.

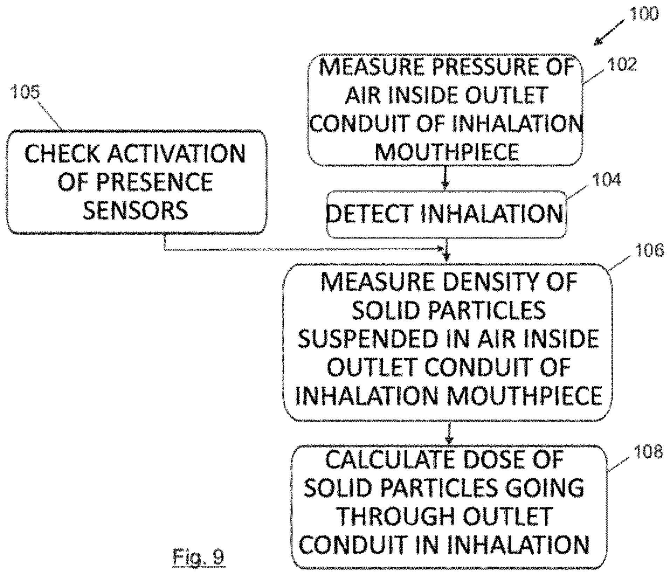

**Figure 9** depicts a flowchart of a dose measuring method 100 for measuring a dose released by both a dry powder inhaler and a pressurized inhaler, according to the present invention. The method, which is carried out by the dose measuring device 1 (Figures 2-6) or the inhaler 70 (Figures 7-8), comprises the following steps:

- Measuring the pressure of the air 102 inside an outlet conduit 35 of an inhalation mouthpiece (3; 73). The mouthpiece may correspond to the inhalation mouthpiece 3 of the dose measuring device 1 (which is coupled to the free end of the mouthpiece 13 of an inhaler 10 or to the free end of the mouthpiece of a spacing chamber for inhalers), the mouthpiece 73 of an inhaler 70 such as the one depicted in Figure 7, or the mouthpiece of a spacing chamber for inhalers.
- Detecting an inhalation 104 by means of analyzing the pressure of the air inside the outlet conduit 35 of the inhalation mouthpiece (3; 73). The start of an inhalation can be detected, for example, by means of detecting a pressure of the air inside the outlet conduit in a given range (e.g. greater than a given threshold).
- Measuring the density of solid particles suspended in air 106 inside the outlet conduit 35 of the inhalation mouthpiece. In the event that the inhalation mouthpiece includes one or several presence sensors 45, the method may comprise the step of checking 105 the activation of the presence sensors as a necessary prior condition. The step of measuring the density of solid particles suspended in air 106 would thereby only be performed when the inhalation and the activation of all the presence sensors is detected.
- Calculating a dose of solid particles 108 suspended in the inhaled air 39 going through the outlet conduit 35 in the detected inhalation.

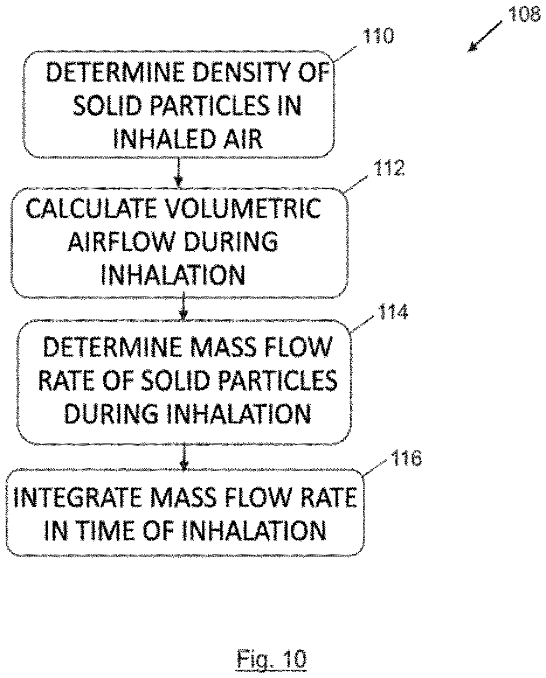

The diagram of **Figure 10** details the calculation of the dose of solid particles 108 going through the outlet conduit 35 in the inhalation according to an embodiment of the dose measuring method 100. The calculation particularly comprises the following steps:

- Determining, by means of the reading of the powder sensor 4, the density (e.g., in $g/m^3$) of solid particles 110 inside the outlet conduit 35 during the inhalation.
- Calculating the volumetric flow rate (e.g., in $m^3/s$) of air 112 going through the outlet conduit 35 during the inhalation using the pressure signal of the barometric pressure sensor 5.
- Determining the mass flow rate (e.g., in g/s) of solid particles 114 going through the outlet conduit 35 during the inhalation.
- Integrating the mass flow rate 116 in the time the inhalation lasts, obtaining the dose (e.g., in g) going through the outlet conduit 35 in the inhalation.

For calculating the dose of solid particles going through the outlet conduit 35 in a given period of time, the same process of Figure 10 is used, in which the density of solid particles, the airflow and the mass flow rate going through the outlet conduit 35 are calculated in a plurality of sampling instants during the period of time, and the mass flow rate is integrated in said period of time for obtaining the dose of solid particles going through the outlet conduit in the period of time. The period of time considered can be the time of the inhalation detected by the barometric pressure sensor 5.

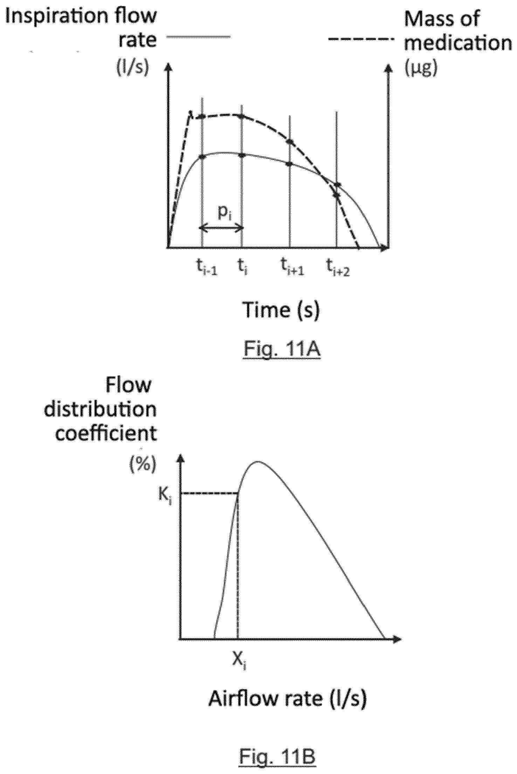

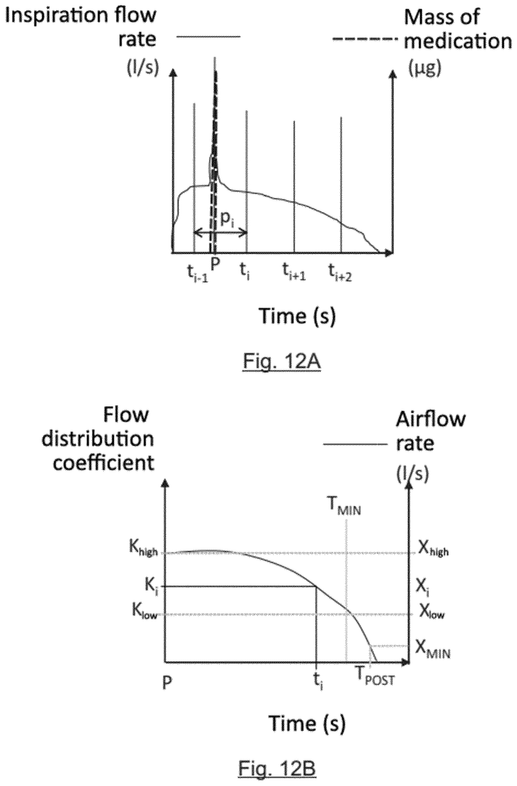

Using the combined information of the powder sensor 4 and of the barometric pressure sensor 5, the dose measuring device 1 is not only capable of calculating the dose of solid particles going through the outlet conduit 35 in the inhalation, but it can also estimate a total dose of medication distributed in the lung in the inhalation. **Figures 11A** and **12A** depict, by way of example, a graph of the inspiration produced during an inhalation in a dry powder inhaler and in a pressurized inhaler, respectively. Both graphs include the inspired airflow rate and the medication mass going through the outlet conduit 35. In the event that the inhalation is performed with a pressurized inhaler (Figure 12A), there is an instant of release of medicinal product P in which the inhaler suddenly releases (usually in a few milliseconds) the entire dose at a high pressure, and a pressure peak represented in Figure 12A is therefore temporarily produced in the instant of release of medicinal product P. In the event that the inhalation is performed with a dry powder inhaler (Figure 11A), said sudden pressure peak generated by the inhaler does not exist.

The control unit 6 calculates the inspired airflow rate and the medication mass in different sampling instants $t_i$. Figures 11A and 12A illustratively represent only four sampling instants; however, during an inhalation that usually lasts for several seconds, the control unit can perform thousands of samplings, because the time between consecutive sampling instants ($t_{i-1}$, $t_i$), sampling period $p_i$, is preferably in the order of milliseconds (e.g. every 10 ms) or even in the order of microseconds. Therefore, the sampling period $p_i$ in Figures 11A and 12A is not represented to scale, but rather is significantly enlarged to enable seeing it in detail.

**Figures 11B** and **12B** show graphs used for estimating the distribution of medication in the lung depending on the inspired airflow rate, and particularly used for obtaining flow distribution coefficients $K_i$ depending on the value of the airflow $X_i$ in the corresponding sampling instants $t_i$. In one embodiment, the control unit 6 is configured for:

- Calculating, using the pressure signal of the barometric pressure sensor 5, the airflow $X_i$ going through the outlet conduit 35 in a plurality of sampling instants $t_i$ during the inhalation.
- Obtaining, for each sampling instant $t_i$, a flow distribution coefficient $K_i$ depending on the value of the airflow $X_i$ in the corresponding sampling instant $t_i$.
- Estimating a total dose of medication distributed in the lung $Z_T$ in the inhalation from the dose of solid particles going through the outlet conduit 35 modified on the basis of the flow distribution coefficients.

Figure 11B illustrates an estimation graph of the distribution of medication in the lung depending on the inspired airflow rate, in the case of an inhalation performed in a dry powder inhaler. The flow distribution coefficient K is represented on the vertical axis. In one embodiment, the flow distribution coefficient K is the percentage of particles going through the outlet conduit 35 estimated to reach the lung, depending on the airflow rate X (in l/s). For lower inspired airflow rates X, it is estimated that a large amount of particles do not reach lung due to a lack of the impulse needed. Likewise, if the inspired airflow rate X is very high, it is estimated that may particles collide with the throat and do not reach lung due to an excess impulse.

The ratio between the flow distribution coefficient K and the airflow rate d largely depends on the characteristics of each inhaler, since each one works with different airflows and has a particle beam with a higher or lower particle distribution ratio. Furthermore, the characteristics of the subject performing the inhalation may also have an effect (factors such as race, weight, sex, height can be considered). The particle size of the medicinal product may also modify said ratio. The ratio between the flow distribution coefficient K and the airflow rate is therefore obtained empirically on the basis of prior studies of the inhaler, for example by means of performing studies *in vivo* with patients performing inhalations for obtaining the precise coefficient of the inhaler. Furthermore, by means of an artificial intelligence algorithm can be deduced if a factor of the person performing the inhalation has to be taken into account (on the basis of the real study with patients, gathering multiple parameters from each individual).

In one embodiment, for the case of an inhalation in a dry powder inhaler (Figures 11A and 11B), the control unit 6 is configured for:

- Calculating, for each sampling instant $t_i$, a dose of solid particles going through the outlet conduit $Y_i$ during a sampling period $p_i$.
- Estimating a dose of medication distributed in the lung $Z_i$ in each sampling instant $t_i$ from the dose of solid particles going through the outlet conduit $Y_i$ modified by the flow distribution coefficient $K_i$ in the corresponding sampling instant $t_i$. Therefore, in the event that the flow distribution coefficient K is the percentage of particles going through the outlet conduit 35 estimated to reach the lung, $Z_i = K_i \cdot Y_i$ is held.
- Calculating the total estimated dose of medication distributed in the lung $Z_T$ in the inhalation by means of the summation of the doses of medication distributed in the lung $Z_i$ in the sampling instants $t_i$ corresponding to the inhalation ($Z_T = \Sigma_i Z_i$).

An inhalation maneuver may include a plurality of successive inhalations and exhalations produced in the inhalation mouthpiece. Therefore, the control unit 6 can be further configured for detecting, by means of analyzing the pressure of the air inside the outlet conduit 35, an inhalation maneuver including a plurality of successive inhalations and exhalations produced in the inhalation mouthpiece; and calculating the total estimated dose of medication distributed in the lung $Z_T$ in the inhalation maneuver by means of the summation of the doses of medication distributed in the lung $Z_i$ in the sampling instants $t_i$ corresponding to the inhalations and the subtraction of the dose of solid particles going through the outlet conduit $Y_i$ during the sampling periods $p_i$ corresponding to the exhalations. The dose of solid particles going through the outlet conduit in the exhalations is thereby discounted, because the direction of the flow rate is opposite the direction of inhalations and said particles do not reach the lungs. Measurements of this type can be used when analyzing the distribution of medication in the lung in the use of a spacing chamber.

Figure 12B illustrates a graph for obtaining flow distribution coefficients $K_i$ in the case of an inhalation performed in a pressurized inhaler. Namely, the graph of Figure 12B allows obtaining a post-release flow distribution coefficient $K_{POST}$ from the flow distribution coefficients $K_i$ in sampling instants $t_i$ after the corresponding release of medicinal product (instant of release of medicinal product P). According to one embodiment, the control unit 6 is configured for:

- Detecting at least one release of medicinal product (instant of release of medicinal product P) during the inhalation. Each release of medicinal product can be detected, for example, by means of detecting a dose of solid particles going through the outlet conduit $Y_i$ during a sampling period $p_i$ greater than a given threshold and/or by means of detecting an airflow $X_i$ going through the outlet conduit 35 greater than a given threshold.
- Obtaining, for each release of medicinal product, a post-release flow distribution coefficient $K_{POST}$ from the flow distribution coefficients $K_i$ in sampling instants $t_i$ after the corresponding release of medicinal product (i.e. after the corresponding instant of release of medicinal product P).
- Calculating the total estimated dose of medication distributed in the lung $Z_T$ in the inhalation from the dose of solid particles going through the outlet conduit $Y_T$ in each release of medicinal product modified by the corresponding post-release flow distribution coefficient $K_{POST}$. In one embodiment, the post-release flow distribution coefficient $K_{POST}$ represents the percentage of particles going through the outlet conduit 35 estimated to reach the lung; that is, $Z_T = K_{POST} \cdot Y_T$.

In one embodiment, the post-release flow distribution coefficient $K_{POST}$ is obtained by means of comparing the flow distribution coefficients $K_i$ with an allowable flow range ($K_{low}$-$K_{high}$), which is determined by a higher airflow $X_{high}$ and a lower airflow $X_{low}$. $K_{high}$ and $K_{low}$ demarcate the acceptable flow range. The control unit 6 detects deviations of the flow distribution coefficients $K_i$ with respect to the allowable flow range ($K_{low}$-$K_{high}$), and obtains the post-release flow distribution coefficient $K_{POST}$ from the duration and/or intensity of the detected deviations.

In one embodiment, the control unit 6 is further configured for:

- Obtaining, for each release of medicinal product, a duration of the post-release inhalation ($T_{POST}$) measured from the release of medicinal product until a new release of medicinal product or until the airflow ($X_i$) going through the outlet conduit (35) is less than a given flow threshold ($X_{MIN}$).
- Obtaining, for each release of medicinal product, a time distribution coefficient $C_T$ by means of comparing the corresponding duration of the post-release inhalation $T_{POST}$ with an inhalation threshold $T_{MIN}$. In Figure 12B, the acceptable timeline marked by $T_{MIN}$ is the minimum inhalation time without penalization (for example, 7 seconds of inhalation may be regarded as an acceptable time).
- Calculating the total estimated dose of medication distributed in the lung $Z_T$ in the inhalation from the dose of solid particles going through the outlet conduit $Y_T$ in each release of medicinal product further modified by the corresponding time distribution coefficient $C_T$. Therefore, in the event that both the post-release flow distribution coefficient $K_{POST}$ and the time distribution coefficient $C_T$ represent percentage-wise contributions to the particles going through the outlet conduit 35 estimated to reach the lung, $Z_T = K_{POST} \cdot C_T \cdot Y_T$ would hold.

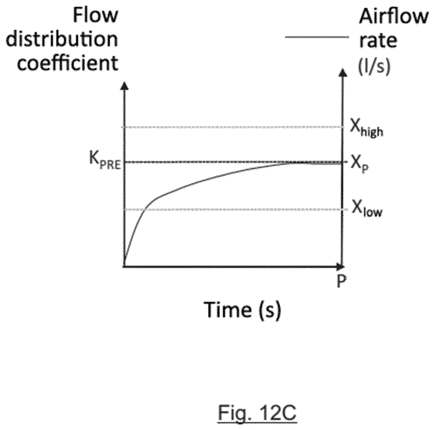

**Figure 12C** depicts a graph for obtaining a pre-release flow distribution coefficient $K_{PRE}$ from the airflow rate conditions in the instants prior to the release of medicinal product (i.e. before the instant of release of medicinal product P). In one embodiment, said pre-release conditions are considered to also contribute to the distribution of the medicinal product in the lungs. To that end, the control unit 6 is preferably configured for:

- Calculating, for each release of medicinal product, the pre-release airflow $X_P$ going through the outlet conduit 35 at the time of the corresponding release of medicinal product, preferably in an instant prior to the corresponding release of medicinal product. Said moment is preferably calculated in the sampling time $t_i$ immediately prior (or even subsequent) to the instant of release of medicinal product P, although another sampling time $t_i$ close to the instant of release of medicinal product P or a mean of several measurements performed in different sampling times $t_i$ close to the instant of release of medicinal product P could be considered.
- Obtaining, for each release of medicinal product, a pre-release flow distribution coefficient $K_{PRE}$ depending on the corresponding value of the pre-release airflow $X_P$.
- Calculating the total estimated dose of medication distributed in the lung $Z_T$ in the inhalation from the dose of solid particles going through the outlet conduit $Y_T$ in each release of medicinal product further modified by the corresponding pre-release flow distribution coefficient $K_{PRE}$. Therefore, if it is considered that both the post-release flow distribution coefficient $K_{POST}$ and the pre-release flow distribution coefficient $K_{PRE}$ represent percentage-wise contributions to the particles going through the outlet conduit 35 estimated to reach the lung, $Z_T = K_{POST} \cdot K_{PRE} \cdot Y_T$ would hold. If, furthermore, the time distribution coefficient $C_T$, $Z_T$ is furthermore considered, it would be calculated as follows: $Z_T = K_{POST} \cdot K_{PRE} \cdot C_T \cdot Y_T$.

Therefore, everything that does not meet optimal (post-release and pre-release) airflow conditions and minimum inhalation time ($T_{MIN}$) conditions, it will have a correction factor (always less than 1), so the total released medication measured $Y_T$ will be multiplied for estimating how much finally reaches the lung.

The dose measuring device 1 is thereby capable of checking the quality of the inhalation in the pressurized inhalers. If the patient remains during the post-release inhalation within the acceptable flow range, a base correction factor will be applied. If the time of inhalation is less than what it should be, a time correction factor, which is independent of the flow correction factor, will be applied. If in the inhalation is not within an acceptable flow range, how much time it was outside the range and how much it departed from the range is calculated, and with that a flow correction factor, which will not modify the time correction factor, will be obtained.

The device can also independently calculate the effect of pre-release (represented by $K_{PRE}$) and the effect of post-release (represented by $K_{POST}$ and $C_T$), and then combine both results. The effect of pre-release considers how much medication collides in the oropharynx, and therefore cannot be inhaled. The post-inhalation effect measures the quality of the inhalation for determining how much of the available medication really reaches the lung.

By way of example, assuming a pre-release airflow of 30 l/min, only 80 % of the delivered medication is available due to colliding with the oropharynx. Therefore, if 100 μg have been delivered, only 80 μg will be available for inhaling. The calculations of the effect post-release with the time correction ($C_T$) and flow correction ($K_{POST}$) must be carried out on the 80 μg available after the pre-release, not on the initial 100 μg. If it is considered that with an inhalation the flow of which is always in range ($X_{low}$-$X_{high}$) and a duration which reaches the stipulated minimum time ($T_{MIN}$) 90 % of the medication reaches the lung, it would mean that the deposition in the lungs was 72 μg. If the inhalation is not perfect, it would be necessary to introduce the time correction factor $C_T$ or flow correction factor $K_{POST}$ and apply it to the inhalation estimated as being perfect. If, for example, the quality of the inhalation was a second shorter than that needed and 10 % of the time it had a flow rate between 10 % and 20 % lower than the allowable range, for 0.5 seconds the time penalization would be an additional 5 % and the flow penalization an additional 15 %.

This would cause the final deposition to be 70 % (10 % which is lost even that it is done perfectly, 5 % time penalization, 15 % flow penalization). Therefore, the initial 100 μg are used to start which, due to the pre-release flow are reduced to an effective maximum of 80 μg, and only 70 % of it would reach the lung, so the medication reaching the lung would be 54 μg.

The device may also comprise at least one presence sensor (implemented by means of at least one pressure sensor, at least one light sensor, at least one impedance sensor or a combination thereof) coupled to the outer face of the inhalation mouthpiece and configured for detecting contact of the mouth of a user with the inhalation mouthpiece, such that the control unit 6 records the detected inhalation when all the presence sensors detect contact of the mouth of a user during the inhalation. If only some but not all presence sensors are activated, it would be impossible to ensure that the medication is suitably dispensed, and therefore it would not be possible to estimate the distribution of the medication since it would be impossible to know if the patient has correctly placed the device in the mouth or if the patient received any medication. This prevents false inhalations or accidental discharges, for example the inhaler is carried in a pocket, from being counted.

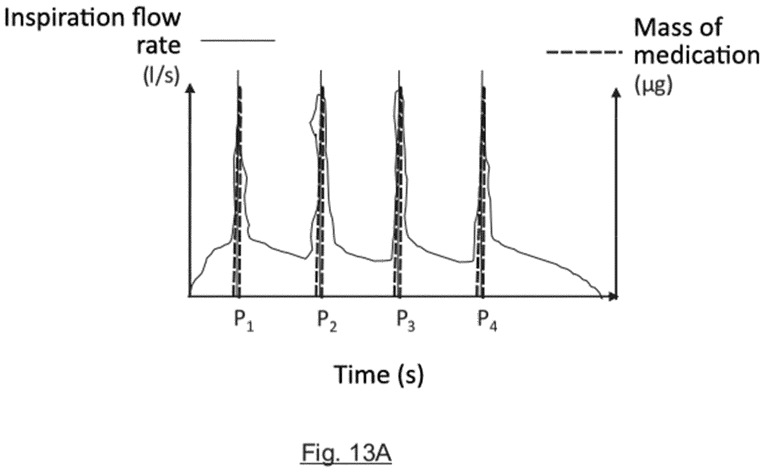

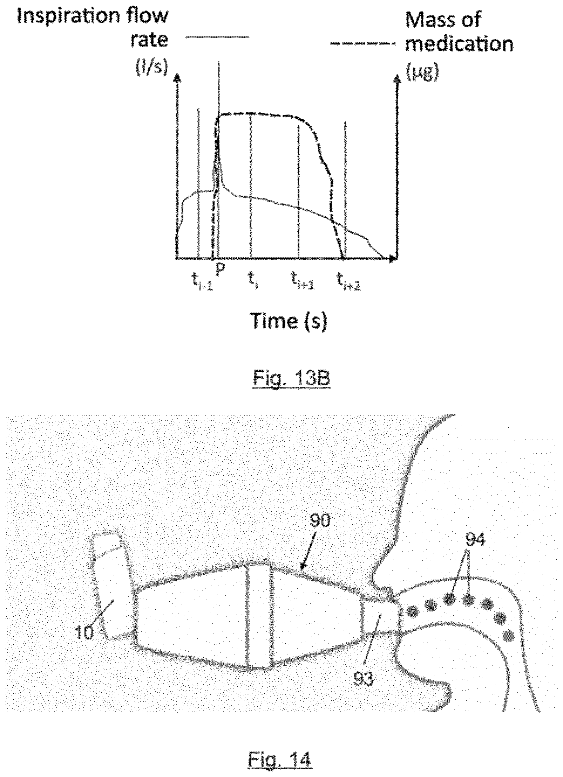

**Figures 13A** and **13B** show, respectively, the particular case of pressurized inhalers with more than one release of medicinal product or with a prolonged release of medicinal product.

In the example of Figure 13A, there are several instants of release of medicinal product P occurring during one and the same inhalation of the user. The calculation of the dose of medicinal product is the same as that explained for a single release (Figure 12A), where for each instant of release of medicinal product $P_i$ there is calculated a flow correction factor prior to the inhalation ($K_{PREi}$) and a post-inhalation correction factor calculated from the flow factor ($K_{POSTi}$) and the time factor ($C_{Ti}$). In each release of medicinal product $P_i$ the medication released by the inhaler (i.e. the dose of solid particles going through the outlet conduit $Y_{Ti}$ in each release of medicinal product) is measured, because it may not be the same in each release. Once the dose estimated to reach the lung is calculated for each release of medicinal product, the total dose of medication distributed in the lung $Z_T$ is obtained as the summation of the doses for each release.

Therefore, according to the example of Figure 13A, there are four instants of release of medicinal product ($P_1$, $P_2$, $P_3$, $P_4$). The dose of medication distributed in the lung $Z_i$ in each of the releases $P_i$ is calculated as:

$$Z_i = K_{POSTi} \cdot K_{PREi} \cdot C_{Ti} \cdot Y_{Ti}.$$

And the total dose of medication distributed in the lung $Z_T$ is calculated as:

$$Z_T = \sum_i Z_i.$$

With respect to the example of Figure 13B, corresponding to a pressurized inhaler with a release of medicinal product prolonged over time (that is, it occurs for several seconds instead of the usual few milliseconds), the calculation is the same as in normal pressurized inhalers, but it is not necessary to take into account only the release of medicinal product in an instant P, but also the dose measured in the all the sampling instants $t_i$ until the dose of solid particles going through the outlet conduit $Y_i$ during a sampling period $p_i$ is zero.

As previously indicated, the inhalation mouthpiece 3 of the dose measuring device 1 can also be coupleable to the free end of the mouthpiece of a spacing chamber for inhalers, using corresponding fixing means. In another embodiment, the inhalation mouthpiece is an integral part of a spacing chamber for inhalers. **Figure 14** shows a spacing chamber 90 which incorporates during manufacture the powder sensor 4 and the barometric pressure sensor 5, installed in the mouthpiece 93 of the spacing chamber 90. The spacing chamber 90 is in turn coupled to an inhaler 10. The dose measuring device 1, through the powder sensor 4 and the barometric pressure sensor installed in the mouthpiece 93 of the spacing chamber 90, calculates the dose of solid particles 94 going through the outlet conduit of the mouthpiece 93 in the detected inhalation, and it is furthermore capable of estimating what amount effectively reaches the lungs considering the flow distribution coefficients ($K_i$) and/or the time distribution coefficients $C_T$ explained in Figures 11 and 12.

## Claims

1.  A dose measuring device for an inhaler, comprising:

    an inhalation mouthpiece (3; 73) comprising an outlet conduit (35) for the passage of inhaled air (39);
    a powder sensor (4) integrated inside the inhalation mouthpiece (3; 73) and configured for providing a reading of the powder density in the inhaled air (39) inside the outlet conduit (35) of the inhalation mouthpiece (3; 73);
    a barometric pressure sensor (5) configured for measuring the pressure of the air inside the outlet conduit (35);
    a control unit (6) configured for:

    detecting an inhalation produced in the inhalation mouthpiece (3; 73) by means of analyzing the pressure of the air inside the outlet conduit (35);
    the dose measuring device being **characterised in that** the control unit is configured for :
    calculating, using the combined information of the powder sensor (4) and the pressure signal of the barometric pressure sensor (5), a dose of solid particles suspended in the inhaled air (39) going through the outlet conduit (35) in the detected inhalation;
    wherein for calculating the dose of solid particles going through the outlet conduit (35) in a period of time, the control unit (6) is configured for:

    - determining, by means of the reading of the powder sensor (4), the density of solid particles inside the outlet conduit (35) in a plurality of sampling instants ($t_i$) during the period of time;
    - calculating the airflow going through the outlet conduit (35) in said plurality of sampling instants ($t_i$) using the pressure signal of the barometric pressure sensor (5);
    - determining the mass flow rate of solid particles going through the outlet conduit (35) in said plurality of sampling instants ($t_i$); and
    - integrating the mass flow rate in said period of time.

**2.** The device according to claim 1, wherein the control unit (6) is configured for:

calculating, using the pressure signal of the barometric pressure sensor (5), the airflow ($X_i$) going through the outlet conduit (35) in a plurality of sampling instants ($t_i$) during the inhalation;

obtaining, for each sampling instant ($t_i$), a flow distribution coefficient ($K_i$) depending on the value of the airflow ($X_i$) in the corresponding sampling instant ($t_i$);

estimating a total dose of medication distributed in the lung ($Z_T$) in the inhalation from the dose of solid particles going through the outlet conduit (35) modified on the basis of the flow distribution coefficients.

**3.** The device according to claim 2, wherein the control unit (6) is configured for:

calculating, for each sampling instant ($t_i$), a dose of solid particles going through the outlet conduit ($Y_i$) during a sampling period ($p_i$);

estimating a dose of medication distributed in the lung ($Z_j$) in each sampling instant ($t_i$) from the dose of solid particles going through the outlet conduit ($Y_i$) modified by the flow distribution coefficient ($K_i$) in the corresponding sampling instant ($t_i$);

calculating the estimated total dose of medication distributed in the lung ($Z_T$) in the inhalation by means of the summation of the doses of medication distributed in the lung ($Z_j$) in the sampling instants ($t_i$) corresponding to the inhalation.

**4.** The device according to claim 3, wherein the control unit (6) is configured for:

detecting, by means of analyzing the pressure of the air inside the outlet conduit (35), an inhalation maneuver including a plurality of successive inhalations and exhalations produced in the inhalation mouthpiece (3; 73);

calculating the total estimated dose of medication distributed in the lung ($Z_T$) in the inhalation maneuver by means of the summation of the doses of medication distributed in the lung ($Z_j$) in the sampling instants ($t_i$) corresponding to the inhalations and the subtraction of the dose of solid particles going through the outlet conduit ($Y_i$) during the sampling periods ($p_i$) corresponding to the exhalations.

**5.** The device according to claim 2, wherein the control unit (6) is configured for:

detecting at least one release of medicinal product during the inhalation;

obtaining, for each release of medicinal product, a post-release flow distribution coefficient ($K_{POST}$) from the flow distribution coefficients ($K_i$) in sampling instants ($t_i$) after the corresponding release of medicinal product;

calculating the total estimated dose of medication distributed in the lung ($Z_T$) in the inhalation from the dose of solid particles going through the outlet conduit ($Y_T$) in each release of medicinal product modified by the corresponding post-release flow distribution coefficient ($K_{POST}$).

**6.** The device according to claim 5, wherein the control unit (6) is configured for:

obtaining, for each release of medicinal product, a duration of the post-release inhalation ($T_{POST}$) measured from the release of medicinal product until a new release of medicinal product or until the airflow ($X_i$) going through the outlet conduit (35) is less than a given flow threshold ($X_{MIN}$);

obtaining, for each release of medicinal product, a time distribution coefficient ($C_T$) by means of comparing the corresponding duration of the post-release inhalation ($T_{POST}$) with an inhalation threshold ($T_{MIN}$);

calculating the total estimated dose of medication distributed in the lung ($Z_T$) in the inhalation from the dose of solid particles going through the outlet conduit ($Y_T$) in each release of medicinal product further modified by the corresponding time distribution coefficient ($C_T$).

**7.** The device according to any of claims 5 to 6, wherein the control unit (6) is configured for:

calculating, for each release of medicinal product, the pre-release airflow ($X_P$) going through the outlet conduit (35) at the time of the corresponding release of medicinal product;

obtaining, for each release of medicinal product, a pre-release flow distribution coefficient ($K_{PRE}$) depending on the corresponding value of the pre-release airflow ($X_P$);

calculating the total estimated dose of medication distributed in the lung ($Z_T$) in the inhalation from the dose of solid particles going through the outlet conduit ($Y_T$) in each release of medicinal product further modified by the corresponding pre-release flow distribution coefficient ($K_{PRE}$).

**8.** The device according to any of the preceding claims, comprising at least one presence sensor (45) coupled to the outer face of the inhalation mouthpiece (3; 73) and configured for detecting contact of the mouth of a user with the inhalation mouthpiece (3; 73);

and wherein the control unit (6) is configured for recording the inhalation detected when the at least one presence sensor (45) detects contact of the mouth of a user during the inhalation.

9. An inhaler comprising a dose measuring device according to any of claims 1 to 8, wherein the inhalation mouthpiece (73) is an integral part of the inhaler (70).

10. A spacing chamber for inhalers comprising a dose measuring device according to any of claims 1 to 8, wherein the inhalation mouthpiece is an integral part of the spacing chamber.

11. A dose measuring method in inhalers, comprising:

measuring, by means of a barometric pressure sensor (5), the pressure of the air (102) inside an outlet conduit (35) of an inhalation mouthpiece (3; 73);
detecting an inhalation (104) by means of analyzing the pressure of the air inside the outlet conduit (35);
obtaining, by means of a powder sensor (4), a reading of the powder density in the inhaled air (39) inside the outlet conduit (35) of the inhalation mouthpiece (3; 73);
calculating, using the combined information of the powder sensor (4) and of the barometric pressure sensor (5), a dose of solid particles (108) suspended in the inhaled air (39) going through the outlet conduit (35) in the detected inhalation; wherein the calculation of the dose of solid particles going through the outlet conduit (35) in a period of time comprises:

- determining, by means of the reading of the powder sensor (4), the density of solid particles (110) inside the outlet conduit (35) in a plurality of sampling instants ($t_i$) during the period of time;
- calculating the airflow (112) going through the outlet conduit (35) in said plurality of sampling instants ($t_i$) using the pressure signal of the barometric pressure sensor (5);
- determining the mass flow rate of solid particles (114) going through the outlet conduit (35) in said plurality of sampling instants (tj); and
- integrating the mass flow rate (116) in said period of time.

12. The method according to claim 11, comprising:

calculating, using the pressure of the air measured inside the outlet conduit (35), the airflow ($X_i$) going through the outlet conduit (35) in a plurality of sampling instants ($t_i$) during the inha-

lation;
obtaining, for each sampling instant ($t_i$), a flow distribution coefficient ($K_i$) depending on the value of the airflow ($X_i$) in the corresponding sampling instant ($t_i$);
estimating a total dose of medication distributed in the lung ($Z_T$) in the inhalation from the dose of solid particles going through the outlet conduit (35) modified on the basis of the flow distribution coefficients.

13. The method according to claim 12, comprising:

calculating, for each sampling instant ($t_i$), a dose of solid particles going through the outlet conduit ($Y_i$) during a sampling period ($p_i$);
estimating a dose of medication distributed in the lung (Zj) in each sampling instant ($t_i$) from the dose of solid particles going through the outlet conduit ($Y_i$) modified by the flow distribution coefficient ($K_i$) in the corresponding sampling instant ($t_i$);
calculating the estimated total dose of medication distributed in the lung ($Z_T$) in the inhalation by means of the summation of the doses of medication distributed in the lung (Zj) in the sampling instants ($t_i$) corresponding to the inhalation.

14. The method according to claim 13, comprising:

detecting, by means of analyzing the pressure of the air inside the outlet conduit (35), an inhalation maneuver including a plurality of successive inhalations and exhalations produced in the inhalation mouthpiece (3; 73);
calculating the total estimated dose of medication distributed in the lung ($Z_T$) in the inhalation maneuver by means of the summation of the doses of medication distributed in the lung (Zj) in the sampling instants ($t_i$) corresponding to the inhalations and the subtraction of the dose of solid particles going through the outlet conduit ($Y_i$) during the sampling periods ($p_i$) corresponding to the exhalations.

15. The method according to claim 12, comprising:

detecting at least one release of medicinal product during the inhalation;
obtaining, for each release of medicinal product, a post-release flow distribution coefficient ($K_{POST}$) from the flow distribution coefficients ($K_i$) in sampling instants (ti) after the corresponding release of medicinal product;
calculating the total estimated dose of medication distributed in the lung ($Z_T$) in the inhalation from the dose of solid particles going through the outlet conduit ($Y_T$) in each release of medicinal product modified by the corresponding post-release flow distribution coefficient ($K_{POST}$).

**Patentansprüche**

1. Vorrichtung zur Dosismessung für einen Inhalator, umfassend:

   ein Inhalationsmundstück (3; 73), umfassend eine Auslassleitung (35) für den Durchgang von inhalierter Luft (39);
   einen Pulversensor (4), der in das Inhalationsmundstück (3; 73) integriert und dazu konfiguriert ist, eine Ablesung der Pulverdichte in der inhalierten Luft (39) innerhalb der Auslassleitung (35) des Inhalationsmundstücks (3; 73) bereitzustellen;
   einen barometrischen Drucksensor (5), der dazu konfiguriert ist, den Druck der Luft innerhalb der Auslassleitung (35) zu messen;
   eine Steuereinheit (6), die für Folgendes konfiguriert ist:

   Erkennen einer in dem Inhalationsmundstück (3; 73) erfolgten Inhalation durch Analyse des Drucks der Luft in der Auslassleitung (35);
   wobei die Vorrichtung zur Dosismessung **dadurch gekennzeichnet ist, dass** die Steuereinheit für Folgendes konfiguriert ist:

   Berechnen, unter Verwendung der kombinierten Informationen des Pulversensors (4) und des Drucksignals des barometrischen Drucksensors (5), einer Dosis von festen Teilchen, die in der inhalierten Luft (39) suspendiert sind, die durch die Auslassleitung (35) bei der erkannten Inhalation geht; wobei zum Berechnen der Dosis von festen Teilchen, die durch die Auslassleitung (35) in einem Zeitraum gehen, die Steuereinheit (6) für Folgendes konfiguriert ist:

   - Bestimmen, mittels der Ablesung des Pulversensors (4), der Dichte von festen Teilchen innerhalb der Auslassleitung (35) in einer Vielzahl von Probenahmezeitpunkten ($t_i$) während des Zeitraums;
   - Berechnen des Luftstroms, der durch die Auslassleitung (35) in der Vielzahl von Probenahmezeitpunkten ($t_i$) geht, unter Verwendung des Drucksignals des barometrischen Drucksensors (5);
   - Bestimmen des Massendurchflusses von festen Teilchen, die durch die Auslassleitung (35) in der Vielzahl von Probenahmezeitpunkten ($t_i$) gehen; und
   - Integrieren des Massendurchflusses in dem Zeitraum.

2. Vorrichtung gemäß Anspruch 1, wobei die Steuereinheit (6) für Folgendes konfiguriert ist:

   Berechnen, unter Verwendung des Drucksignals des barometrischen Drucksensors (5), des Luftstroms ($X_i$), der durch die Auslassleitung (35) in einer Vielzahl von Probenahmezeitpunkten ($t_i$) während der Inhalation geht;
   Erhalten, für jeden Probenahmezeitpunkt ($t_i$), eines Flussverteilungskoeffizienten ($K_i$) in Abhängigkeit von dem Wert des Luftstroms ($X_i$) in dem entsprechenden Probenahmezeitpunkt ($t_i$);
   Abschätzen einer Gesamtdosis des in der Lunge ($Z_T$) verteilten Medikaments bei der Inhalation aus der Dosis der festen Teilchen, die durch die Auslassleitung (35) gehen, modifiziert basierend auf den Flussverteilungskoeffizienten.

3. Vorrichtung gemäß Anspruch 2, wobei die Steuereinheit (6) für Folgendes konfiguriert ist:

   Berechnen, für jeden Probenahmezeitpunkt ($t_i$), einer Dosis von festen Teilchen, die durch die Auslassleitung ($Y_i$) während eines Probenahmezeitraums ($p_i$) gehen;
   Abschätzen einer Dosis eines in der Lunge ($Z_i$) verteilten Medikaments zu jedem Probenahmezeitpunkt ($t_i$) aus der Dosis von festen Teilchen, die durch die Auslassleitung ($Y_i$) gehen, modifiziert durch den Flussverteilungskoeffizienten ($K_i$) zu dem entsprechenden Probenahmezeitpunkt ($t_i$);
   Berechnen der abgeschätzten Gesamtdosis des in der Lunge ($Z_T$) verteilten Medikaments bei der Inhalation mittels der Summierung der Dosen des in der Lunge ($Z_i$) verteilten Medikaments zu den Probenahmezeitpunkten ($t_j$), die der Inhalation entsprechen.

4. Vorrichtung gemäß Anspruch 3, wobei die Steuereinheit (6) für Folgendes konfiguriert ist:

   Erkennen eines Inhalationsmanövers, beinhaltend eine Vielzahl von aufeinanderfolgenden Inhalationen und Exhalationen, die in dem Inhalationsmundstück (3; 73) erfolgen, durch Analyse des Drucks der Luft innerhalb der Auslassleitung (35);
   Berechnen der abgeschätzten Gesamtdosis des im Inhalationsmanöver in der Lunge ($Z_T$)

verteilten Medikaments mittels der Summierung der Dosen des in der Lunge ($Z_i$) verteilten Medikaments zu den Probenahmezeitpunkten (tj), die den Inhalationen entsprechen, und der Subtraktion der Dosis der festen Teilchen, die während der Probenahmezeiträume ($p_i$), die den Exhalationen entsprechen, durch die Auslassleitung ($Y_i$) gehen.

5. Vorrichtung gemäß Anspruch 2, wobei die Steuereinheit (6) für Folgendes konfiguriert ist:

Erkennen mindestens einer Freisetzung eines Arzneimittels während der Inhalation;
Erhalten eines Flussverteilungskoeffizienten nach der Freisetzung ($K_{POST}$) aus den Flussverteilungskoeffizienten ($K_i$) zu den Probenahmezeitpunkten ($t_i$) nach der entsprechenden Freisetzung des Arzneimittels für jede Freisetzung des Arzneimittels;
Berechnen der abgeschätzten Gesamtdosis des in der Lunge ($Z_T$) verteilten Medikaments bei der Inhalation aus der Dosis der festen Teilchen, die durch die Auslassleitung ($Y_T$) gehen, bei jeder Freisetzung des Arzneimittels, modifiziert durch den entsprechenden Flussverteilungskoeffizienten nach der Freisetzung ($K_{POST}$).

6. Vorrichtung gemäß Anspruch 5, wobei die Steuereinheit (6) für Folgendes konfiguriert ist:

Erhalten, für jede Freisetzung eines Arzneimittels, einer Dauer der Inhalation nach der Freisetzung ($T_{POST}$), gemessen von der Freisetzung des Arzneimittels bis zu einer neuen Freisetzung des Arzneimittels oder bis der Luftstrom ($X_i$), der durch die Auslassleitung (35) geht, kleiner ist als ein gegebener Flussschwellenwert ($X_{MIN}$);
Erhalten, für jede Freisetzung eines Arzneimittels, eines Zeitverteilungskoeffizienten ($C_T$) durch den Vergleich der entsprechenden Dauer der Inhalation nach der Freisetzung ($T_{POST}$) mit einem Inhalationsschwellenwert ($T_{MIN}$);
Berechnen der abgeschätzten Gesamtdosis des in der Lunge ($Z_T$) verteilten Medikaments bei der Inhalation aus der Dosis der festen Teilchen, die durch die Auslassleitung ($Y_T$) gehen, bei jeder Freisetzung des Arzneimittels, ferner modifiziert durch den entsprechenden Zeitverteilungskoeffizienten ($C_T$).

7. Vorrichtung gemäß einem der Ansprüche 5 bis 6, wobei die Steuereinheit (6) für Folgendes konfiguriert ist:

Berechnen, für jede Freisetzung eines Arzneimittels, des Luftstroms vor der Freisetzung ($X_P$), der durch die Auslassleitung (35) zum Zeitpunkt der entsprechenden Freisetzung eines Arzneimittels geht;
Erhalten, für jede Freisetzung eines Arzneimittels, eines Flussverteilungskoeffizienten vor der Freisetzung ($K_{PRE}$), der von dem entsprechenden Wert des Luftstroms vor der Freisetzung ($X_P$) abhängt;
Berechnen der abgeschätzten Gesamtdosis des in der Lunge ($Z_T$) verteilten Medikaments bei der Inhalation aus der Dosis der festen Teilchen, die durch die Auslassleitung ($Y_T$) gehen, bei jeder Freisetzung des Arzneimittels, ferner modifiziert durch den entsprechenden Flussverteilungskoeffizienten vor der Freisetzung ($K_{PRE}$).

8. Vorrichtung gemäß einem der vorhergehenden Ansprüche, umfassend mindestens einen Anwesenheitssensor (45), der mit der Außenseite des Inhalationsmundstücks (3; 73) gekoppelt ist und dazu konfiguriert ist, den Kontakt des Mundes eines Benutzers mit dem Inhalationsmundstück (3; 73) zu erkennen;
und wobei die Steuereinheit (6) dazu konfiguriert ist, die Inhalation aufzuzeichnen, die erkannt wird, wenn der mindestens eine Anwesenheitssensor (45) den Kontakt des Mundes eines Benutzers während der Inhalation erkennt.

9. Inhalator, umfassend eine Vorrichtung zur Dosismessung gemäß einem der Ansprüche 1 bis 8, wobei das Inhalationsmundstück (73) ein integraler Teil des Inhalators (70) ist.

10. Abstandskammer für Inhalatoren, umfassend eine Vorrichtung zur Dosismessung gemäß einem der Ansprüche 1 bis 8, wobei das Inhalationsmundstück ein integraler Teil der Abstandskammer ist.

11. Verfahren zur Dosismessung in Inhalatoren, umfassend:

Messen des Drucks der Luft (102) in einer Auslassleitung (35) eines Inhalationsmundstücks (3; 73) mittels eines barometrischen Drucksensors (5);
Erkennen einer Inhalation (104) durch Analyse des Drucks der Luft in der Auslassleitung (35);
Erhalten einer Ablesung der Pulverdichte in der inhalierten Luft (39) innerhalb der Auslassleitung (35) des Inhalationsmundstücks (3; 73) mittels eines Pulversensors (4);
Berechnen, unter Verwendung der kombinierten Informationen des Pulversensors (4) und des barometrischen Drucksensors (5), einer Dosis von festen Teilchen (108), die in der inhalierten Luft (39) suspendiert sind, die durch die Auslassleitung (35) bei der erkannten Inhalation geht; wobei das Berechnen der Dosis von festen Teilchen, die durch die Auslassleitung (35) in einem Zeitraum gehen, Folgendes umfasst:

- Bestimmen, mittels der Ablesung des Pulversensors (4), der Dichte von festen Teilchen (110) innerhalb der Auslassleitung (35) in einer Vielzahl von Probenahmezeitpunkten ($t_i$) während des Zeitraums;
- Berechnen des Luftstroms (112), der durch die Auslassleitung (35) in der Vielzahl von Probenahmezeitpunkten ($t_i$) geht, unter Verwendung des Drucksignals des barometrischen Drucksensors (5);
- Bestimmen des Massendurchflusses von festen Teilchen (114), die durch die Auslassleitung (35) in der Vielzahl von Probenahmezeitpunkten ($t_i$) gehen; und
- Integrieren des Massendurchflusses (116) in dem Zeitraum.

12. Verfahren gemäß Anspruch 11, umfassend:

Berechnen, unter Verwendung des in der Auslassleitung (35) gemessenen Luftdrucks, des Luftstroms ($X_i$), der durch die Auslassleitung (35) in einer Vielzahl von Probenahmezeitpunkten ($t_i$) während der Inhalation geht;

Erhalten, für jeden Probenahmezeitpunkt ($t_i$), eines Flussverteilungskoeffizienten ($K_i$) in Abhängigkeit von dem Wert des Luftstroms ($Xi$) in dem entsprechenden Probenahmezeitpunkt ($t_i$);

Abschätzen einer Gesamtdosis des in der Lunge ($Z_T$) verteilten Medikaments bei der Inhalation aus der Dosis der festen Teilchen, die durch die Auslassleitung (35) gehen, modifiziert basierend auf den Flussverteilungskoeffizienten.

13. Verfahren gemäß Anspruch 12, umfassend:

Berechnen, für jeden Probenahmezeitpunkt ($t_j$), einer Dosis von festen Teilchen, die durch die Auslassleitung ($Y_i$) während eines Probenahmezeitraums ($p_i$) gehen;

Abschätzen einer Dosis eines in der Lunge ($Z_i$) verteilten Medikaments zu jedem Probenahmezeitpunkt ($t_i$) aus der Dosis von festen Teilchen, die durch die Auslassleitung ($Y_i$) gehen, modifiziert durch den Flussverteilungskoeffizienten ($K_i$) zu dem entsprechenden Probenahmezeitpunkt ($t_i$);

Berechnen der abgeschätzten Gesamtdosis des in der Lunge ($Z_T$) verteilten Medikaments bei der Inhalation mittels der Summierung der Dosen des in der Lunge ($Z_i$) verteilten Medikaments zu den Probenahmezeitpunkten ($t_j$), die

der Inhalation entsprechen.

14. Verfahren gemäß Anspruch 13, umfassend:

Erkennen eines Inhalationsmanövers, beinhaltend eine Vielzahl von aufeinanderfolgenden Inhalationen und Exhalationen, die in dem Inhalationsmundstück (3; 73) erfolgen, durch Analyse des Drucks der Luft innerhalb der Auslassleitung (35);

Berechnen der abgeschätzten Gesamtdosis des im Inhalationsmanöver in der Lunge ($Z_T$) verteilten Medikaments mittels der Summierung der Dosen des in der Lunge ($Z_i$) verteilten Medikaments zu den Probenahmezeitpunkten ($t_j$), die den Inhalationen entsprechen, und der Subtraktion der Dosis der festen Teilchen, die während der Probenahmezeiträume ($p_i$), die den Exhalationen entsprechen, durch die Auslassleitung ($Y_i$) gehen.

15. Verfahren gemäß Anspruch 12, umfassend:

Erkennen mindestens einer Freisetzung eines Arzneimittels während der Inhalation;

Erhalten eines Flussverteilungskoeffizienten nach der Freisetzung ($K_{POST}$) aus den Flussverteilungskoeffizienten ($K_i$) zu den Probenahmezeitpunkten ($t_i$) nach der entsprechenden Freisetzung des Arzneimittels für jede Freisetzung des Arzneimittels;

Berechnen der abgeschätzten Gesamtdosis des in der Lunge ($Z_T$) verteilten Medikaments bei der Inhalation aus der Dosis der festen Teilchen, die durch die Auslassleitung ($Y_T$) gehen, bei jeder Freisetzung des Arzneimittels, modifiziert durch den entsprechenden Flussverteilungskoeffizienten nach der Freisetzung ($K_{POST}$).

**Revendications**

1. Dispositif de mesure de la dose pour un inhalateur, comprenant :

un embout d'inhalation (3 ; 73) comprenant un conduit de sortie (35) pour le passage de l'air inhalé (39) ;

un capteur de poudre (4) intégré dans l'embout d'inhalation (3 ; 73) et configuré pour fournir une lecture de la densité de la poudre dans l'air inhalé (39) à l'intérieur du conduit de sortie (35) de l'embout d'inhalation (3 ; 73) ;

un capteur de pression barométrique (5) configuré pour mesurer la pression de l'air à l'intérieur du conduit de sortie (35) ;

une unité de commande (6) configurée pour :

détection d'une inhalation produite dans l'embout d'inhalation (3 ; 73) au moyen de l'analyse de la pression de l'air à l'intérieur du conduit de sortie (35) ;

le dispositif de mesure de la dose est **caractérisé en ce que** l'unité de commande est configurée pour :

calculer, en utilisant les informations combinées du capteur de poudre (4) et le signal de pression du capteur de pression barométrique (5), une dose de particules solides en suspension dans l'air inhalé (39) passant par le conduit de sortie (35) lors de l'inhalation détectée ; dans lequel, pour calculer la dose de particules solides passant par le conduit de sortie (35) dans une période de temps, l'unité de commande (6) est configurée pour :

- déterminer, au moyen de la lecture du capteur de poudre (4), la densité des particules solides à l'intérieur du conduit de sortie (35) au cours d'une pluralité d'instants d'échantillonnage ($t_i$) pendant la période de temps ;
- calculer le débit d'air passant par le conduit de sortie (35) dans ladite pluralité d'instants d'échantillonnage ($t_i$) à l'aide du signal de pression du capteur de pression barométrique (5) ;
- déterminer le débit massique des particules solides passant par le conduit de sortie (35) dans ladite pluralité d'instants d'échantillonnage ($t_i$) ; et
- intégrer le débit massique au cours de cette période de temps.

2. Le dispositif selon la revendication 1, dans lequel l'unité de commande (6) est configurée pour :

calculer, à l'aide du signal de pression du capteur de pression barométrique (5), le débit d'air ($X_i$) passant par le conduit de sortie (35) à plusieurs instants d'échantillonnage ($t_i$) au cours de l'inhalation ;

obtenir, pour chaque instant d'échantillonnage ($t_i$), un coefficient de répartition du débit ($K_i$) en fonction de la valeur du débit d'air ($X_i$) à l'instant d'échantillonnage correspondant ($t_i$) ;

l'estimation d'une dose totale de médicament distribuée dans le poumon ($Z_T$) lors de l'inhalation à partir de la dose de particules solides passant par le conduit de sortie (35) modifié sur la base des coefficients de distribution du flux.

3. Le dispositif selon la revendication 2, dans lequel l'unité de commande (6) est configurée pour :

calculer, pour chaque instant d'échantillonnage ($t_i$), une dose de particules solides passant par le conduit de sortie ($Y_i$) au cours d'une période d'échantillonnage ($p_i$) ;

estimer une dose de médicament distribuée dans le poumon ($Z_i$) à chaque instant d'échantillonnage ($t_i$) à partir de la dose de particules solides passant par le conduit de sortie ($Y_i$) modifiée par le coefficient de distribution du flux ($K_i$) à l'instant d'échantillonnage correspondant ($t_i$) ;

calculer la dose totale estimée de médicament distribuée dans les poumons ($Z_T$) lors de l'inhalation au moyen de la somme des doses de médicament distribuées dans les poumons ($Z_i$) aux instants d'échantillonnage ($t_i$) correspondant à l'inhalation.

4. Le dispositif selon la revendication 3, dans lequel l'unité de commande (6) est configurée pour :

détecter, par l'analyse de la pression de l'air à l'intérieur du conduit de sortie (35), une manoeuvre d'inhalation comprenant une pluralité d'inhalations et d'exhalations successives produites dans l'embout d'inhalation (3 ; 73) ;

calculer la dose totale estimée de médicament distribuée dans le poumon ($Z_T$) lors de la manoeuvre d'inhalation au moyen de la somme des doses de médicament distribuées dans le poumon ($Z_i$) lors des instants d'échantillonnage ($t_i$) correspondant aux inhalations et de la soustraction de la dose de particules solides passant par le conduit de sortie ($Y_i$) lors des périodes d'échantillonnage ($p_i$) correspondant aux exhalations.

5. Le dispositif selon la revendication 2, dans lequel l'unité de commande (6) est configurée pour :

détecter au moins une libération de médicament pendant l'inhalation ;

obtenir, pour chaque libération de médicament, un coefficient de distribution du débit après libération ($K_{POST}$) à partir des coefficients de distribution du débit ($K_i$) aux instants d'échantillonnage ($t_i$) après la libération correspondante du médicament ;

calculer la dose totale estimée de médicament distribuée dans le poumon ($Z_T$) lors de l'inhalation à partir de la dose de particules solides passant par le conduit de sortie ($Y_T$) dans chaque libération de médicament modifiée par le coefficient de distribution du flux après libération correspondant ($K_{POST}$).

6. Le dispositif selon la revendication 5, dans lequel l'unité de commande (6) est configurée pour :

obtenir, pour chaque libération de médicament, une durée de l'inhalation post-libération ($T_{POST}$) mesurée à partir de la libération du médicament jusqu'à une nouvelle libération du médicament ou jusqu'à ce que le débit d'air ($X_i$) passant par le conduit de sortie (35) soit inférieur à un seuil de débit donné ($X_{MIN}$) ;

obtenir, pour chaque libération de médicament, un coefficient de distribution temporelle ($C_T$) en comparant la durée correspondante de l'inhalation après libération ($T_{POST}$) avec un seuil d'inhalation ($T_{MIN}$) ;

calculer la dose totale estimée de médicament distribuée dans le poumon ($Z_T$) lors de l'inhalation à partir de la dose de particules solides passant par le conduit de sortie ($Y_T$) dans chaque libération de médicament, modifiée en outre par le coefficient de distribution temporelle ($C_T$) correspondant.

7.  Le dispositif selon les revendications 5 ou 6, dans lequel l'unité de commande (6) est configurée pour :

    calculer, pour chaque libération de médicament, le débit d'air de prélibération ($X_P$) passant par le conduit de sortie (35) au moment de la libération correspondante du médicament ;

    obtenir, pour chaque libération de médicament, un coefficient de distribution du débit avant libération ($K_{PRE}$) en fonction de la valeur correspondante du débit d'air avant libération ($X_P$);

    calculer la dose totale estimée de médicament distribuée dans le poumon ($Z_T$) lors de l'inhalation à partir de la dose de particules solides passant par le conduit de sortie ($Y_T$) dans chaque libération de médicament, modifiée en outre par le coefficient de distribution du débit avant libération correspondant ($K_{PRE}$).

8.  Le dispositif selon l'une quelconque des revendications précédentes, comprenant au moins un capteur de présence (45) couplé à la face extérieure de l'embout d'inhalation (3 ; 73) et configuré pour détecter le contact de la bouche d'un utilisateur avec l'embout d'inhalation (3 ; 73) ;

    et dans lequel l'unité de commande (6) est configurée pour enregistrer l'inhalation détectée lorsqu'au moins un capteur de présence (45) détecte le contact de la bouche d'un utilisateur pendant l'inhalation.

9.  Inhalateur comprenant un dispositif de mesure de la dose selon l'une des revendications 1 à 8, dans lequel l'embout d'inhalation (73) fait partie intégrante de l'inhalateur (70).

10. Chambre d'espacement pour inhalateurs comprenant un dispositif de mesure de la dose selon l'une des revendications 1 à 8, dans laquelle l'embout d'inhalation fait partie intégrante de la chambre d'espacement.

11. Procédé de mesure de la dose dans les inhalateurs, comprenant :

    mesurer, au moyen d'un capteur de pression barométrique (5), la pression de l'air (102) à l'intérieur d'un conduit de sortie (35) d'un embout d'inhalation (3 ; 73) ;

    détecter une inhalation (104) par l'analyse de la pression de l'air à l'intérieur du conduit de sortie (35) ;

    obtenir, au moyen d'un capteur de poudre (4), une lecture de la densité de la poudre dans l'air inhalé (39) à l'intérieur du conduit de sortie (35) de l'embout d'inhalation (3 ; 73) ;

    calculer, en utilisant les informations combinées du capteur de poudre (4) et du capteur de pression barométrique (5), une dose de particules solides (108) en suspension dans l'air inhalé (39) passant par le conduit de sortie (35) lors de l'inhalation détectée ; le calcul de la dose de particules solides passant par le conduit de sortie (35) au cours d'une période de temps comprend :

    - déterminer, au moyen de la lecture du capteur de poudre (4), la densité des particules solides (110) à l'intérieur du conduit de sortie (35) dans une pluralité d'instants d'échantillonnage ($t_i$) au cours de la période de temps ;
    - calculer le débit d'air (112) passant par le conduit de sortie (35) dans ladite pluralité d'instants d'échantillonnage ($t_i$) en utilisant le signal de pression du capteur de pression barométrique (5) ;
    - déterminer le débit massique des particules solides (114) passant par le conduit de sortie (35) dans ladite pluralité d'instants d'échantillonnage ($t_i$) ; et
    - intégrer le débit massique (116) au cours de cette période de temps.

12. Procédé selon la revendication 11, comprenant :

    calculer, à l'aide de la pression de l'air mesurée à l'intérieur du conduit de sortie (35), le débit d'air ($X_i$) passant par le conduit de sortie (35) à plusieurs instants d'échantillonnage ($t_i$) pendant l'inhalation ;

    obtenir, pour chaque instant d'échantillonnage ($t_i$), un coefficient de répartition du débit ($K_i$) en fonction de la valeur du débit d'air ($X_i$) à l'instant d'échantillonnage correspondant ($t_i$) ;

    l'estimation d'une dose totale de médicament distribuée dans le poumon ($Z_T$) lors de l'inhalation à partir de la dose de particules solides passant par le conduit de sortie (35) modifié sur la base des coefficients de distribution du flux.

13. Procédé selon la revendication 12, comprennant :

calculer, pour chaque instant d'échantillonnage $(t_i)$, une dose de particules solides passant par le conduit de sortie $(Y_i)$ au cours d'une période d'échantillonnage $(p_i)$ ;
estimer une dose de médicament distribuée dans le poumon $(Z_i)$ à chaque instant d'échantillonnage $(t_i)$ à partir de la dose de particules solides passant par le conduit de sortie $(Y_i)$ modifiée par le coefficient de distribution du flux $(K_i)$ à l'instant d'échantillonnage correspondant $(t_i)$ ;
calculer la dose totale estimée de médicament distribuée dans les poumons $(Z_T)$ lors de l'inhalation au moyen de la somme des doses de médicament distribuées dans les poumons $(Z_i)$ aux instants d'échantillonnage $(t_i)$ correspondant à l'inhalation.

14. Procédé selon la revendication 13, comprennant :

détecter, par l'analyse de la pression de l'air à l'intérieur du conduit de sortie (35), une manoeuvre d'inhalation comprenant une pluralité d'inhalations et d'exhalations successives produites dans l'embout d'inhalation (3 ; 73) ;
calculer la dose totale estimée de médicament distribuée dans le poumon $(Z_T)$ lors de la manoeuvre d'inhalation au moyen de la somme des doses de médicament distribuées dans le poumon $(Z_i)$ lors des instants d'échantillonnage $(t_i)$ correspondant aux inhalations et de la soustraction de la dose de particules solides passant par le conduit de sortie $(Y_i)$ lors des périodes d'échantillonnage $(p_i)$ correspondant aux exhalations.

15. Procédé selon la revendication 12, comprennant :

détecter au moins une libération de médicament pendant l'inhalation ;
obtenir, pour chaque libération de médicament, un coefficient de distribution du débit après libération $(K_{POST})$ à partir des coefficients de distribution du débit $(K_i)$ aux instants d'échantillonnage $(t_i)$ après la libération correspondante du médicament ;
calculer la dose totale estimée de médicament distribuée dans le poumon $(Z_T)$ lors de l'inhalation à partir de la dose de particules solides passant par le conduit de sortie $(Y_T)$ dans chaque libération de médicament modifiée par le coefficient de distribution du flux après libération correspondant $(K_{POST})$.

Fig. 1

Fig. 2A

Fig. 2B

**Fig. 2C**

**Fig. 2D**

Fig. 2E

Fig. 3

Fig. 4A

Fig. 4B

Fig. 5

60

Dose measuring device 1

Inhalation mouthpiece 3

45 Presence sensor

5 Barometric pressure sensor

4 Powder sensor

17 Sleep mode wake-up signal

Casing 2

6 Control unit

9 Bluetooth module

16 Memory

24

8 DC/DC converter

7 Battery

25

52 Notification/Alert

50

APP

Fig. 6

70

73

Fig. 7A

70

73

Fig. 7B

Fig. 7C

**Dry powder inhaler** <u>70</u>

**Inhalation mouthpiece** <u>73</u>

4 — Powder sensor

5 — Barometric pressure sensor

45 — Presence sensor

17 — Sleep mode wake-up signal

6 — Control unit

Bluetooth module

APP

50

16 — Memory

7 — Battery

8 — DC/DC converter

80

Fig. 8

EP 3 993 858 B1

100

MEASURE PRESSURE OF
AIR INSIDE OUTLET
CONDUIT OF INHALATION
MOUTHPIECE
102

105

CHECK ACTIVATION
OF PRESENCE
SENSORS

DETECT INHALATION
104

MEASURE DENSITY OF
SOLID PARTICLES
SUSPENDED IN AIR INSIDE
OUTLET CONDUIT OF
INHALATION MOUTHPIECE
106

CALCULATE DOSE OF
SOLID PARTICLES GOING
THROUGH OUTLET
CONDUIT IN INHALATION
108

Fig. 9

108

110

DETERMINE DENSITY OF SOLID PARTICLES IN INHALED AIR

112

CALCULATE VOLUMETRIC AIRFLOW DURING INHALATION

114

DETERMINE MASS FLOW RATE OF SOLID PARTICLES DURING INHALATION

116

INTEGRATE MASS FLOW RATE IN TIME OF INHALATION

Fig. 10

Inspiration flow rate (l/s)

Mass of medication (μg)

$p_i$

$t_{i-1}$ $t_i$ $t_{i+1}$ $t_{i+2}$

Time (s)

Fig. 11A

Flow distribution coefficient (%)

$K_i$

$X_i$

Airflow rate (l/s)

Fig. 11B

Fig. 12A

Fig. 12B

Fig. 12C

Fig. 13A

EP 3 993 858 B1

Fig. 13B

Fig. 14

**EP 3 993 858 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018175579 A1